# EUROPEAN PATENT SPECIFICATION

(11) **EP 3 689 311 B1**
(45) Date of publication and mention of the grant of the patent: **08.11.2023**
(21) Application number: 18863683.1
(22) Date of filing: 25.09.2018
(51) Int. Cl.: B29C 65/08, A61F 13/51, A61F 13/49, A61F 13/496

(54) **ELASTIC MEMBER, DISPOSABLE WEARABLE ITEM USING THIS ELASTIC MEMBER, AND METHOD FOR PRODUCING ELASTIC MEMBER**
ELASTISCHES ELEMENT, AM KÖRPER TRAGBARER EINWEGARTIKEL MIT DIESEM ELASTISCHEN ELEMENT UND VERFAHREN ZUR HERSTELLUNG EINES ELASTISCHEN ELEMENTS
ÉLÉMENT ÉLASTIQUE, ARTICLE VESTIMENTAIRE JETABLE UTILISANT CET ÉLÉMENT ÉLASTIQUE, ET PROCÉDÉ DE PRODUCTION D'ÉLÉMENT ÉLASTIQUE

(30) Priority: 27.09.2017 JP 2017187179; 24.11.2017 JP 2017226330
(43) Date of publication of application: 05.08.2020
(73) Proprietor: Daio Paper Corporation, Shikokuchuo-shi, Ehime 799-0492 (JP)
(72) Inventor: SAKAI, Syunsuke, Sakura-shi Tochigi 329-1411 (JP)
(74) Representative: Meissner Bolte Partnerschaft mbB
(86) International application number: PCT/JP2018/035311
(87) International publication number: WO 2019/065574

(56) References cited:
- JP-A- 2015 109 900
- JP-A- 2017 064 130
- JP-A- 2017 064 226
- US-A1- 2017 239 105

## Description

### Technical Field

The present invention relates to an elastic member having a stretchable structure in which an elastic sheet such as an elastic film is interposed between a first sheet layer and a second sheet layer, and a disposable wearing article including this elastic member.

### Background Art

In a disposable wearing article such as a disposable diaper, to improve fitting to a body surface, it is common to impart elasticity to an appropriate place such as around legs or around a waist. As a method of imparting elasticity, a method of attaching an elongated elastic member such as rubber thread in a state of being stretched in a longitudinal direction has been widely adopted. However, in the case of imparting elasticity at a certain width, a mode in which rubber threads are fixed in a state of being arranged side by side with an interval in the width has been adopted. In addition, as a method of obtaining an excellent surface fitting, a method of attaching an elastic sheet in a state of being stretched in a direction of imparting elasticity has been proposed. (For example, see Patent Literature 1 and Patent Literature 2) .

The elastic member including the elastic sheet is obtained when an elastic film is stacked between a first sheet layer and a second sheet layer, and, in a state in which the elastic film is stretched in an stretchable direction, the first sheet layer and the second sheet layer are welded by a plurality of dotted sheet joined portions arranged at intervals in the stretchable direction and a direction orthogonal thereto through joint holes formed in the elastic film. Further, in this elastic member, in a natural length state, as the elastic sheet contracts between the sheet joined portions, the intervals between the sheet joined portions decrease, and pleats are formed to extend in a direction intersecting the stretchable direction between the sheet joined portions in the first sheet layer and the second sheet layer. On the contrary, during stretching, as the elastic sheet stretches between the sheet joined portions, the intervals between the sheet joined portions and the pleats in the first sheet layer and the second sheet layer widen, and elastic stretching is allowed up to a fully unfolded state of the first sheet layer and the second sheet layer. A stretchable region by this elastic sheet is advantageous in that surface fitting is excellent, there is no bonding between the first sheet layer and the second sheet layer, and the elastic sheet, the structure is significantly flexible due to extremely little bonding between the first sheet layer and the second sheet layer, and the joint holes of the elastic sheet also contribute to improvement in air permeability.

On the other hand, since the elasticity and appearance of the elastic member including the elastic sheet change depending on the shape of the sheet joined portions, it is desirable to provide a plurality of sheet joined portions having different shapes depending on the site of the disposable wearing article.

However, when a plurality of sheet joined portions having different shapes is provided, the first sheet layer and the second sheet layer are not welded or are in a state of being peeled by a weak force even though the sheet layers are welded at some sheet joined portions (welding defect) .
Patent literature 3 discloses an elastic member having an elastic sheet stretchable structure in which an elastic sheet is interposed between a first sheet layer made of a nonwoven fabric and a second sheet layer (made of a nonwoven fabric and the first sheet layer and the second sheet layer are welded through joint holes penetrating the elastic sheet at a plurality of sheet joined portions arranged at intervals, wherein a region having the elastic sheet stretchable structure includes a stretchable region that contracts in an stretchable direction by contraction of the elastic sheet and is extensible in the stretchable direction, the region having the elastic sheet stretchable structure includes sheet joined portions having different shapes. Patent literature 3 also discloses an underpants-type disposable article, which comprises such elastic member.
Patent literature 4 discloses an underpants-type disposable diaper, which has outer members 12F and 12B and stretchable regions A2 and A3 disposed adjacent to the inner edges of the side seal portions 12A in the width direction. The stretchable regions A2 and A3 include multiple elongated elastically stretchable members 19 disposed between two sheet layers 12S and 12H at predetermined intervals in the front-back direction and extending in the width direction and the two sheet layers 12S and 12H are welded into sheet bonding sections 20 at positions between the elastically stretchable members 19 in the stretchable regions A2 and A3 adjacent in the front-back direction.
Patent literature 5 discloses an underpants-type disposable diaper with improved fit to the lower part of a gluteal region. This is achieved by means of an outer body, which is formed by stacking an elastic sheet layer between a first sheet layer and a second sheet layer, so that when the elastic sheet layer is extended in a width direction, the first sheet layer and the second sheet layer are joined together directly or indirectly at a large number of dot-like joint portions arranged at intervals left in the width-direction and front-back direction.

### Citation List

### Patent Literature

Patent Literature 1: JP 2016-189932 A
Patent Literature 2: JP 2015-204982 A
Patent Literature 3: JP 2017-064226 A
Patent Literature 4: JP 2017-064130 A
Patent Literature 5: US 2017-239105 A

### Summary of Invention

### Technical Problem

Therefore, a main object of the invention is to make it difficult to cause a welding defect in the case of including the sheet joined portions having different shapes.

### Solution to Problem

An elastic member solving the above-mentioned problem, a disposable wearing article including this elastic member, and a method of manufacturing the elastic member are as follows.

### <First aspect>

An elastic member having an elastic sheet stretchable structure in which an elastic sheet is interposed between a first sheet layer made of a nonwoven fabric and a second sheet layer made of a nonwoven fabric and the first sheet layer and the second sheet layer are welded through joint holes penetrating the elastic sheet at a plurality of sheet joined portions arranged at intervals,
wherein the first sheet layer and the second sheet layer are formed of nonwoven fabrics having a fineness of 0.7 to 6 dtex and a basis weight of 10 to 25 g/m²,
in which a region having the elastic sheet stretchable structure includes a stretchable region that contracts in an stretchable direction by contraction of the elastic sheet and is extensible in the stretchable direction,
the region having the elastic sheet stretchable structure includes sheet joined portions having different shapes,
in all the sheet joined portions in the region having the elastic sheet stretchable structure, a reference bonding diameter **ϕ** is 0.25 to 0.5 mm, and a circumference length of the sheet joined portions is 1 to 15 times a length of a circumference of a circle whose diameter is the reference bonding diameter **ϕ,** and the reference bonding diameter **ϕ** refers to a diameter of a largest inscribed circle inscribed in the outer shape of the sheet joined portion 40.

### (Effect)

As a result of studying the above-mentioned welding defect, the present inventor has found that even though the welding defect when sheet joined portions having different shapes are present is improved by increasing a linear pressure at the time of forming the sheet joined portions (increasing a force pressure in ultrasonic sealing), the improvement is not only limited, but also leads to early wear and breakage of equipment such as an anvil roll, which is not preferable. Therefore, as a result of further studying a cause of the welding defect, it has found that even when sheet joined portions having different shapes are present, it is possible to make the welding defect hardly occur by setting the reference bonding diameter and the circumference length to specific ranges as described above. Here, the reference bonding diameter refers to a diameter of a largest inscribed circle inscribed in the outer shape of the sheet joined portion.

A reason why it is possible to make the welding defect hardly occur by setting the reference bonding diameter and the circumference length to the specific ranges as described above is considered as follows. That is, for the welding, it is necessary that the nonwoven fabric is melted by spreading heat from a center of a heated portion in a radial direction to some extent and over a somewhat sufficient range. To this end, it is preferable that the sheet joined portions have a shape that can include a sufficiently large circle, that is, a reference bonding diameter of a certain degree or more. According to the knowledge of the present inventor, a reference bonding diameter is from 0.25 mm to 0.5 mm. Further, the sheet joined portions having an excessively long or excessively complicated outer shape tend to cause a partial welding defect. From this viewpoint, it is preferable that the circumference length of the sheet joined portions is within the above range. Further, in a case in which a difference in the reference bonding diameter between the sheet joined portions is excessively large, when the linear pressure at the time of forming the sheet joined portions is adjusted to that of a sheet joined portion having a large reference bonding diameter, a welding defect is likely to occur. In addition, when the linear pressure at the time of forming the sheet joined portions is adjusted to that of a sheet joined portion having a small reference bonding diameter, there is concern about leading to early wear or breakage of equipment such as an anvil roll. On the other hand, when the reference bonding diameter is set to a specific range as described above, it is possible to perform welding that is less likely to cause the welding defect with an appropriate pressure.

In the elastic member the first sheet layer and the second sheet layer are formed of nonwoven fabrics having a fineness of 0.7 to 6 dtex and a basis weight of 10 to 25 g/m².

### (Effect)

In order to achieve both suppression of the welding defect and prevention of early wear/breakage of equipment, the nonwoven fabric and the reference bonding diameter in the above ranges are particularly preferable.

### <Second aspect>

The elastic member according to the first aspect,
in which the region having the elastic sheet stretchable structure includes the stretchable region and a non-stretchable region provided on at least one side of the stretchable region in the stretchable direction,
an area ratio of the sheet joined portions in the stretchable region is 0.5 to 1 times an area ratio of the sheet joined portions in the non-stretchable region, and
a boundary between the stretchable region and the non-stretchable region has a shape continuously shifted only to one side in the stretchable direction from one end to the other end thereof.

### (Effect)

By changing the area ratio of the sheet joined portions, it is possible to change an elasticity property. However, when a position at which the area ratio of the sheet joined portions rapidly changes, that is, a boundary between the stretchable region and the non-stretchable region extends along the orthogonal direction orthogonal to the stretchable direction from one end to the other end thereof, a linear pressure rapidly changes at the boundary between the stretchable region and the non-stretchable region during welding. Such a rapid change in linear pressure may lead to early wear or breakage of equipment such as an anvil roll. On the other hand, in the case of the present aspect, there is no rapid change in linear pressure, and thus there is little concern about leading to early wear or breakage of equipment such as an anvil roll.

### <Third aspect>

An underpants-type disposable wearing article including
an integrated outer member from a front body to a back body or outer members separately provided for the front body and the back body,
an inner member attached to an intermediate portion of the outer member in a width direction, the inner member extending to both front and back sides of a crotch portion,
side seal portions in which both side portions of the outer member in the front body and both side portions of the outer member in the back body are bonded to each other, respectively, and
a waist opening and a pair of right and left leg openings,
in which the outer member in the front body and the back body has a lower torso portion which is a range in a front-back direction corresponding to the side seal portions,
the outer member in at least one of the front body and the back body has an intermediate portion located on a center side of the lower torso portion in the front-back direction,
the intermediate portion has edge portion regions along the leg openings,
the outer member having the intermediate portion is an elastic member having an elastic sheet stretchable structure of a first aspect from an inside of the intermediate portion to an inside of the lower torso portion in the front-back direction and between the side seal portions in the width direction so that an stretchable direction of a stretchable region thereof corresponds to the width direction, and
a shape of the sheet joined portions in the edge portion regions along the leg openings is different from a shape of the sheet joined portions in other regions.

### (Effect)

The elastic member described above is suitable for the outer member of the underpants-type disposable wearing article as in the present aspect. In particular, it is preferable that the elastic sheet stretchable structure is provided from the inside of the intermediate portion to the inside of the lower torso portion in the front-back direction and between the side seal portions in the width direction, and the elasticity of edge portions along leg openings is made different from the elasticity of other regions, thereby improving fitting of around-leg portions and other regions. In this case, the shape of the sheet joined portions in the edge portion regions along the leg openings is different from the shape of the sheet joined portions in other regions, and thus there is concern that the welding defect may occur. However, when the reference bonding diameter and the circumference length are set to specific ranges as described above, it is possible to perform welding that is less likely to cause the welding defect with an appropriate pressure as described in the first aspect.

### <Fourth aspect>

The disposable wearing article according to the third aspect,
in which the outer member having the elastic sheet stretchable structure has a non-stretchable region in an intermediate portion in the width direction and a range in the width direction corresponding to a part between the non-stretchable region and the side seal portions is set as the stretchable region,
an area ratio of the sheet joined portions in the stretchable region is 0.5 to 1 times an area ratio of the sheet joined portions in the non-stretchable region, and
a boundary between the stretchable region and the non-stretchable region has a shape continuously shifted only to sides of the side seal portions from an end of a waist opening side to a center of the disposable wearing article in the front-back direction.

### (Effect)

In the underpants-type disposable wearing article, the non-stretchable region is generally provided at the position of this aspect. Therefore, in this case, it is desirable to shift the position of the boundary between the stretchable region and the non-stretchable region in the orthogonal direction as in the second aspect. However, since the boundary between the stretchable region and the non-stretchable region in this case extends in the front-back direction of the underpants-type disposable wearing article, shortening of the stretchable region on the waist opening side is not preferable from a viewpoint of ensuring fitting. Therefore, as in the present aspect, it is preferable that a position of the boundary between the stretchable region and the non-stretchable region is continuously shifted only to sides of the side seal portions from the end on the waist opening side to the center of the disposable wearing article in the front-back direction.

### <Fifth aspect>

A method of manufacturing an elastic member as specified herein above, the method including
a supplying process of interposing an elastic sheet between a first sheet layer and a second sheet layer in a state of being stretched in an MD (machine direction), and
a bonding process of passing the first sheet layer, the second sheet layer, and the elastic sheet interposed therebetween in a stretched state between an anvil roll having a plurality of projections arranged at intervals in a predetermined pattern on an outer peripheral surface and an ultrasonic horn facing the outer peripheral surface of the anvil roll, and welding the first sheet layer and the second sheet layer only at portions interposed between the plurality of projections and the ultrasonic horn to form sheet joined portions,
in which sheet joined portions having different reference bonding diameters are formed by one ultrasonic horn, and
in all the sheet joined portions formed by the one ultrasonic horn, a reference bonding diameter is 0.25 mm to 0.5 mm, , and a circumference length of the sheet joined portions is 1 to 15 times a length of a circumference of a circle whose diameter is the reference bonding diameter.

### (Effect)

In a case in which the sheet joined portions are formed by ultrasonic sealing, the same effect as that of the first aspect is achieved.

### <Sixthaspect>

A method of manufacturing an elastic member, the method including
a supplying process of interposing an elastic sheet between a first sheet layer and a second sheet layer in a state of being stretched in an MD, and
a bonding process of passing the first sheet layer, the second sheet layer, and the elastic sheet interposed therebetween in a stretched state between an anvil roll having a plurality of projections arranged at intervals in a predetermined pattern on an outer peripheral surface and an opposing roll facing the outer peripheral surface of the anvil roll, and welding the first sheet layer and the second sheet layer only at portions interposed between the plurality of projections and the opposing roll by the anvil roll and opposing roll that are heated to form sheet joined portions,
in which sheet joined portions having different reference bonding diameters are formed by a pair of anvil rolls and the opposing roll, and
in all the sheet joined portions formed by the pair of anvil rolls and the opposing roll, a reference bonding diameter is 0.25 to 0.5 mm, and a circumference length of the sheet joined portions is 1 to 15 times a length of a circumference of a circle whose diameter is the reference bonding diameter.

### (Effect)

In a case in which the sheet joined portions are formed by heat sealing, the same effect as that of the first aspect is achieved.

### Advantageous Effects of Invention

According to the invention, there is an advantage that it is difficult to cause a welding defect in the case of including the sheet joined portions having different shapes.

### Brief Description of Drawings

Fig. 1 is a plan view (internal surface side) of an underpants-type disposable diaper in an unfolded state.
Fig. 2 is a plan view (external surface side) of the underpants-type disposable diaper in the unfolded state.
Fig. 3 is a plan view illustrating only a main part of the underpants-type disposable diaper in the unfolded state.
Fig. 4(a) is a cross-sectional view taken along C-C line of Fig. 1, and Fig. 4(b) is a cross-sectional view taken along E-E line of Fig. 1.
Fig. 5 is a cross-sectional view taken along A-A line of Fig. 1.
Fig. 6 is a cross-sectional view taken along B-B line of Fig. 1.
Fig. 7(a) is a plan view of a main part of a stretchable region, Fig. 7(b) is a cross-sectional view taken along D-D line of Fig. 7(a), Fig. 7(c) is a cross-sectional view in a worn state, and Fig. 7(d) is a cross-sectional view in a natural length state.
Fig. 8 is a cross-sectional view schematically illustrating a cross section of a main part of an outer member stretched to some extent.
Fig. 9(a) is a plan view of a main part of a stretchable region, Fig. 9(b) is a cross-sectional view taken along D-D line of Fig. 9(a), Fig. 9(c) is a cross-sectional view in a worn state, and Fig. 9(d) is a cross-sectional view in a natural length state.
Fig. 10 is a plan view illustrating various arrangements of sheet joined portions.
Fig. 11 is a plan view of the stretchable region in the unfolded state.
Fig. 12 is an enlarged plan view illustrating a main part of the stretchable region in the unfolded state.
Fig. 13 is an enlarged plan view illustrating a main part of the stretchable region in the natural length state.
Fig. 14(a) is a cross-sectional view taken along D-D line of Fig. 12, and Fig. 14(b) is a cross-sectional view in the natural length state.
Fig. 15 is a plan view of the stretchable region in the unfolded state.
Fig. 16 is an enlarged plan view illustrating the main part of the stretchable region in the unfolded state.
Fig. 17 is an enlarged plan view illustrating the main part of the stretchable region in the natural length state.
Fig. 18 is a front view illustrating only a main part of the underpants-type disposable diaper.
Fig. 19 is a front view illustrating only a main part of the underpants-type disposable diaper.
Fig. 20 is a front view illustrating only a main part of the underpants-type disposable diaper.
Fig. 21 is a schematic view of an ultrasonic sealing device.
Fig. 22 is an enlarged plan view illustrating a part Q of Fig. 2.
Fig. 23 is an enlarged plan view illustrating the part Q of Fig. 2.
Fig. 24 is a plan view illustrating various shapes of the sheet joined portions.

### Description of Embodiments

Hereinafter, a detailed description will be given of an elastic member, a disposable wearing article, and a method of manufacturing the elastic member based on an example of an underpants-type disposable diaper illustrated in accompanying drawings. Incidentally, a dotted pattern portion in a cross-sectional view illustrates bonding means such as a hotmelt adhesive.

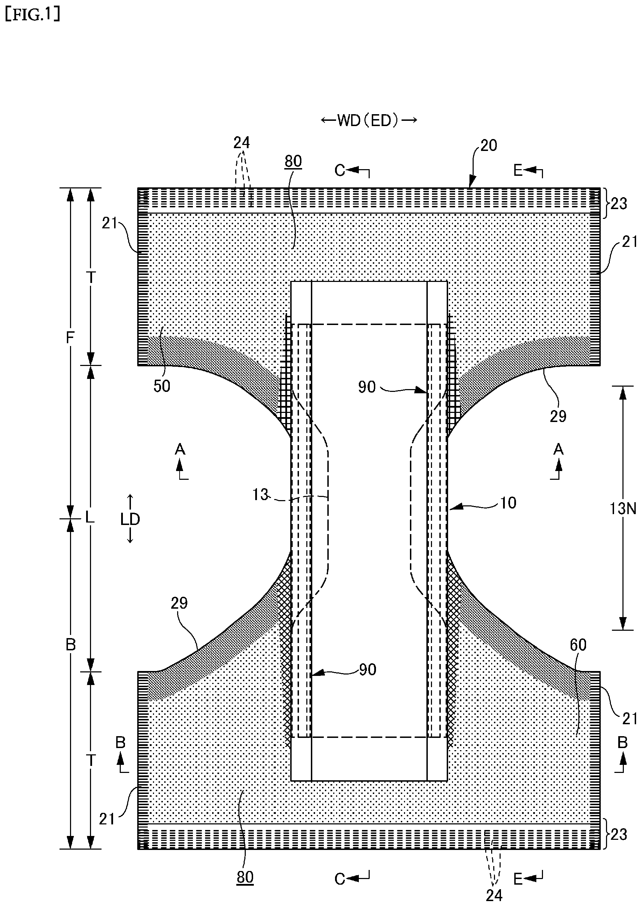

Fig. 1 to Fig. 6 illustrate the underpants-type disposable diaper. A reference character LD (longitudinal direction) denotes a front-back direction, and a reference character WD denotes a width direction. The underpants-type disposable diaper (hereinafter also simply referred to as a diaper) includes an outer member 20 forming a front body F and a back body B, and an inner member 10 fixed to and integrated with an inner surface of the outer member 20, and the inner member 10 is formed by interposing an absorbent body 13 between a liquid pervious top sheet 11 and a liquid impervious sheet 12. In manufacturing, after a back surface of the inner member 10 is bonded to the inner surface (upper surface) of the outer member 20 by bonding means such as a hotmelt adhesive, the inner member 10 and the outer member 20 are folded at a center in the front-back direction LD (longitudinal direction) corresponding to a boundary between the front body F and the back body B, and both side portions thereof are bonded to each other by thermal welding or the hotmelt adhesive to form side seal portions 21, thereby obtaining the underpants-type disposable diaper in which a waist opening and a pair of right and left leg openings are formed.

### (Structure example of inner member)

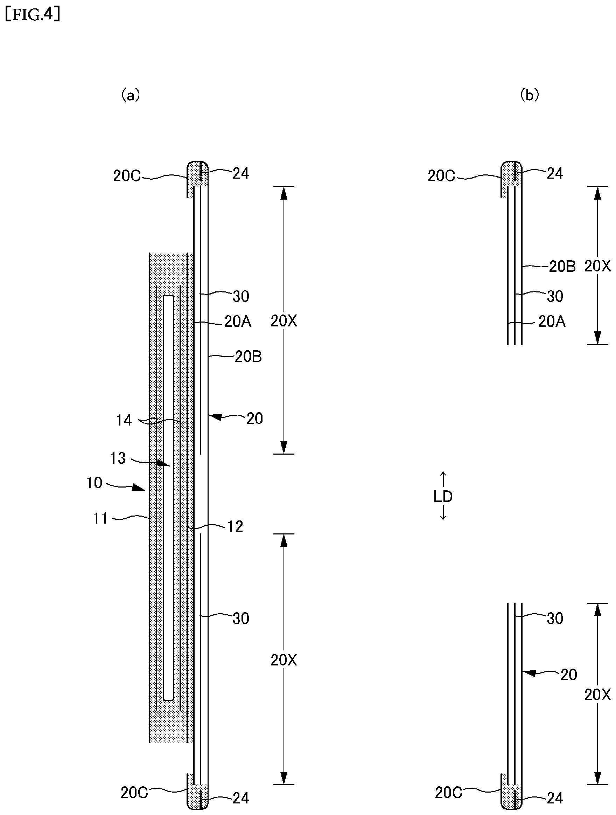

As illustrated in Fig. 4 to Fig. 6, the inner member 10 has a structure in which the absorbent body 13 is interposed between the top sheet 11 and the liquid impervious sheet 12 made of polyethylene, etc. and absorbs and holds excretion fluid passing through the top sheet 11. A planar shape of the inner member 10 is not particularly limited. However, a substantially rectangular shape is generally adopted as illustrated in Fig. 1.

As the top sheet 11 that covers the front surface side (skin side) of the absorbent body 13, a perforated or non-perforated nonwoven fabric, a porous plastic sheet, etc. is preferably used. As a material fiber constituting the nonwoven fabric, it is possible to adopt a regenerated fiber such as rayon and cupra or a natural fiber such as cotton in addition to a polyolefin-based synthetic fiber such as polyethylene or polypropylene, a polyester-based synthetic fiber, a polyamide-based synthetic fiber, etc., and it is possible to use a nonwoven fabric obtained by an appropriate processing method such as a spunlace method, a spunbond method, a thermal bond method, a meltblown method, a needle punch method, etc.

As the liquid impervious sheet 12 covering the back surface side (non-skin contact side) of the absorbent body 13, a liquid impervious plastic sheet such as polyethylene or polypropylene may be used. In particular, a sheet having a moisture penetration property may be preferably used from a viewpoint of preventing stuffiness. Examples thereof include a microporous sheet obtained by melt-kneading an inorganic filler in a polyolefin resin such as polyethylene or polypropylene to form a sheet, and then stretching the sheet in a uniaxial or biaxial direction.

As the absorbent body 13, it is possible to use a known one, for example, a pulp fiber stack, an assembly of filaments of cellulose acetate, etc., or a nonwoven fabric-based body mixed with a high-absorbent polymer as necessary or fixed. To hold the shape and the polymer, the absorbent body 13 can be wrapped in a package sheet 14 having a liquid pervious and liquid retaining property such as crepe paper as necessary.

The absorbent body 13 is formed into a substantially hourglass shape having a narrower portion 13N narrower than both front and back sides at a crotch portion. A size of the narrower portion 13N can be determined as appropriate. A length of the narrower portion 13N in the front-back direction can be set to about 20 to 50% of a maximum length of the diaper, and a width of a narrowest portion thereof can be set to about 40 to 60% of a maximum width of the absorbent body 13. In the case of having such a narrower portion 13N, when the planar shape of the inner member 10 is substantially rectangular, non-absorbent body side portions 17 not having the absorbent body 13 are formed at a portion corresponding to the narrower portion 13N of the absorbent body 13 in the inner member 10.

The liquid impervious sheet 12 is folded back to the back surface side on both sides of the absorbent body 13 in the width direction together with the top sheet 11. As this liquid impervious sheet 12, it is desirable to use an opaque sheet so that brown color of excreta or urine is not seen. As opacification, a pigment or a filler such as calcium carbonate, titanium oxide, zinc oxide, white carbon, clay, talc, or barium sulfate added to plastic and formed into a film is preferably used.

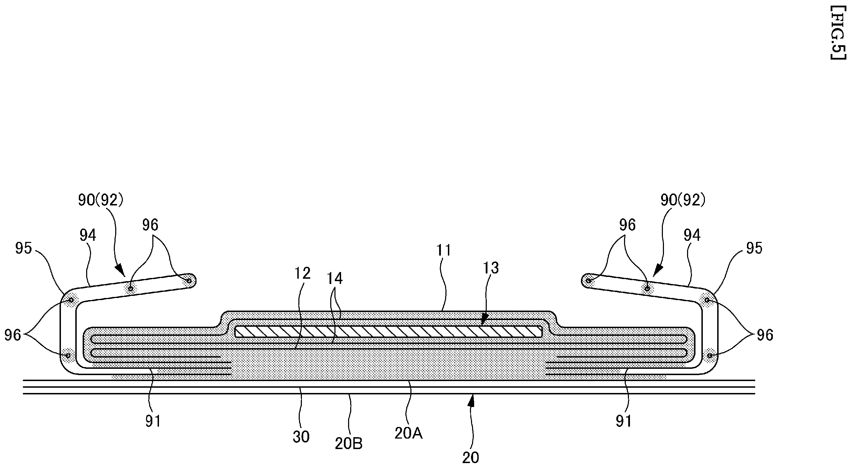

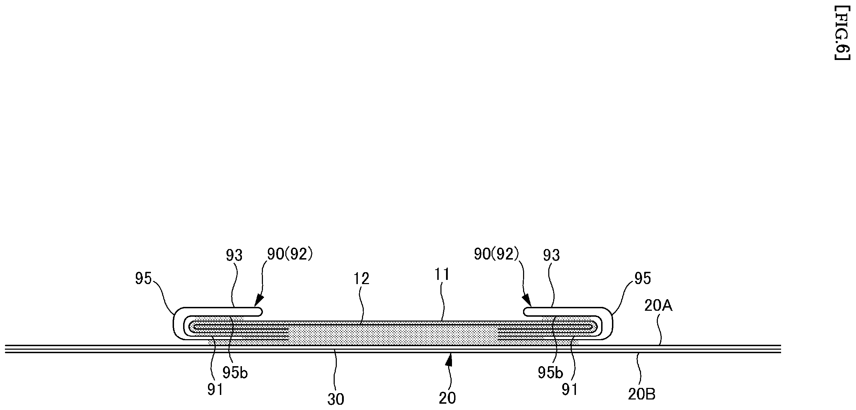

Three-dimensional gathers 90 fit around the legs are formed on both side portions of the inner member 10. As illustrated in Fig. 5 and Fig. 6, each of the three-dimensional gathers 90 includes a fixed portion 91 fixed to a side portion of the back surface of the inner member 10, a main unit section 92 extending from the fixed portion 91 up to a side portion of the front surface of the inner member 10 through a side of the inner member 10, a fallen portion 93 formed by front and back end portions of the main unit section 92 fixed to the side portion of the front surface of the inner member 10 (top sheet 11 in the illustrated example) in a fallen state using a hotmelt adhesive 95b, etc., and a free portion 94 formed between parts of the fallen portion 93 which are not fixed. Each of these portions is formed of a gather sheet 95 that is a duplicate sheet obtained by folding a sheet such as a nonwoven fabric. The gather sheet 95 is attached over the entire inner member 10 in the front-back direction, the fallen portion 93 is provided on the front side and the back side of each of the non-absorbent body side portions 17, and the free portion 94 extends to both the front and back sides of the non-absorbent body side portion 17. In addition, between the double gather sheets 95, gather elastic members 96 are disposed at tip portions of the free portion. As illustrated in Fig. 5, the gather elastic members 96 are for raising the free portion 94 by an elastic contraction force in a product state.

A fixing structure of the gather elastic members 96 and the gather sheets 95 is not particularly limited. For example, as in an example illustrated in Fig. 5 and Fig. 6, it is possible to adopt a structure described in the following. In portions other than the fallen portion 93, the gather elastic members 96 are attached and fixed to the gather sheets 95 through a hotmelt adhesive at positions of the gather elastic members 96, and facing surfaces of the gather sheets 95 are bonded to each other. However, in the fallen portion 93, the hotmelt adhesive is not present at the positions of the gather elastic members 96. Therefore, the gather elastic members 96 and the gather sheets 95 are not attached to each other, and the facing surfaces of the gather sheets 95 are not bonded to each other at positions having the gather elastic members 96.

As the gather elastic members 96, it is possible to use normally used materials such as polystyrene-based rubber, polyolefin-based rubber, polyurethane-based rubber, polyester-based rubber, polyurethane, polyethylene, polystyrene, styrene-butadiene copolymer, silicone, polyester, etc. In addition, to make it difficult to see from the outside, it is preferable that a fineness is set to 925 dtex or less, a tension is set to 150 to 350%, and an interval is set to 7.0 mm or less. Incidentally, as the gather elastic members 96, it is possible to use a tape-like member having a certain width in addition to an elongated member as in the illustrated example.

As a material fiber constituting the gather sheets 95 described above, similarly to the top sheet 11, it is possible to adopt a regenerated fiber such as rayon or cupra or a natural fiber such as cotton in addition to a polyolefin-based synthetic fiber such as polyethylene or polypropylene, a polyester-based synthetic fiber, a polyamide-based synthetic fiber, etc., and it is possible to use a nonwoven fabric obtained by an appropriate processing method such as a spunbond method, a thermal bond method, a meltblown method, a needle punch method, etc. However, in particular, in order to prevent stuffiness, it is preferable to use a nonwoven fabric that suppresses a basis weight and has excellent air permeability. Further, with regard to the gather sheets 95, to prevent passage of urine, etc., prevent a rash, and enhance a feel to a skin (dry feeling), it is preferable to use a water repellent nonwoven fabric coated with a silicone-based, paraffin metal-based, or alkylchromic chloride-based water repellent agent, etc.

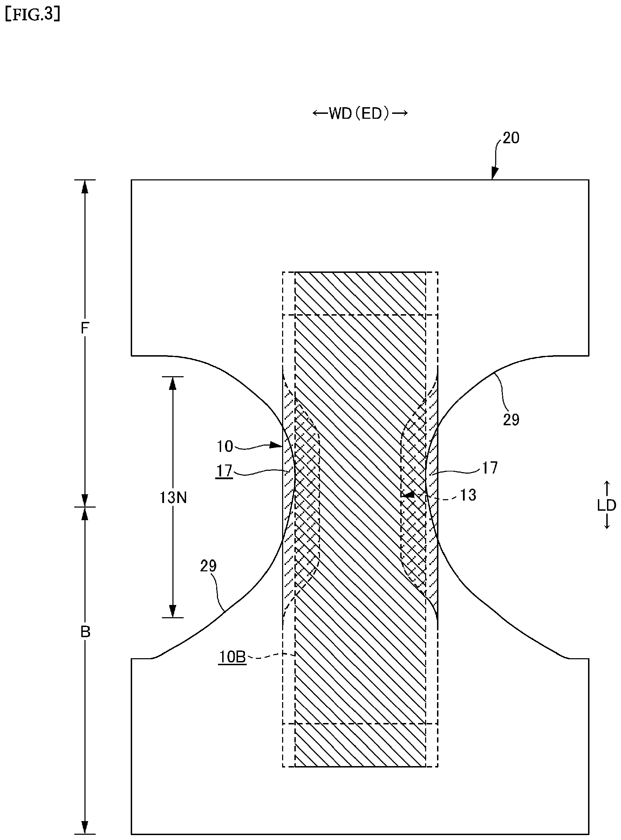

As illustrated in Fig. 3 to Fig. 6, the back surface of the inner member 10 is bonded to the inner surface of the outer member 20 by a hotmelt adhesive, etc. in an inner/outer fixing region 10B (shaded region). The inner/outer fixing region 10B may be determined as appropriate and may correspond to almost the entire inner member 10 in a width direction WD. However, it is preferable that both ends in the width direction are not fixed to the outer member 20.

### (Structure example of outer member)

The outer member 20 includes at least the lower torso portion T of the front body F and the lower torso portion T of the back body B, and further includes an intermediate portion L corresponding to a range in the front-back direction between the lower torso portion T of the front body F and the lower torso portion T of the back body B in the illustrated example. Referring to the outer member 20, as in the illustrated example, in a crotch portion, side edges of the outer member 20 may be located on a central side from side edges of the inner member 10 in the width direction or located on an outer side thereof in the width direction.

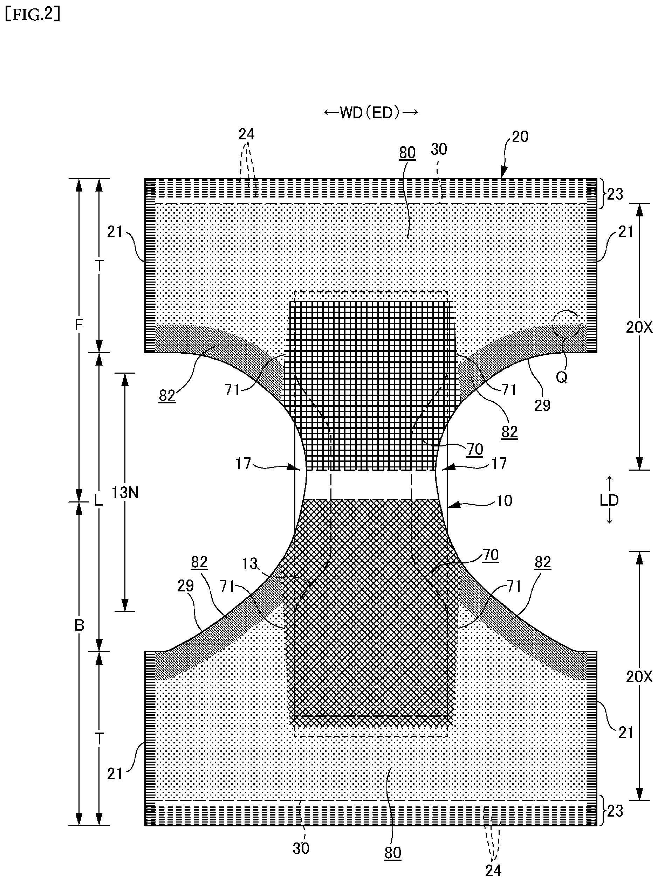

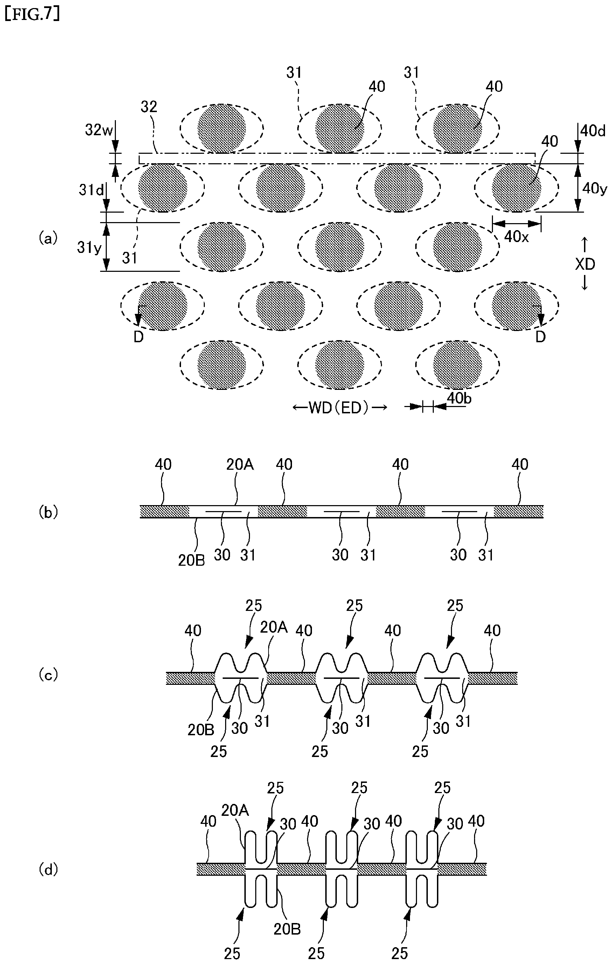

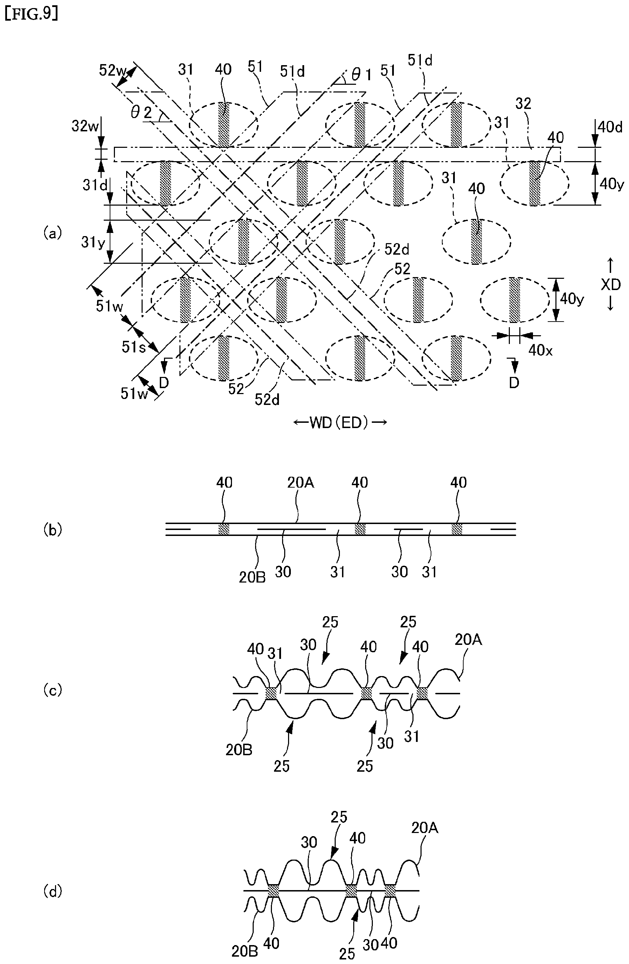

Further, the outer member 20 of the illustrated example has an elastic sheet stretchable structure 20X in which an elastic sheet 30 is interposed between the first sheet layer 20A and the second sheet layer 20B as illustrated in Fig. 2 and Fig. 4 to Fig. 6 except for a middle of the intermediate portion L in the front-back direction and the first sheet layer 20A and the second sheet layer 20B are bonded through joint holes 31 penetrating the elastic sheet 30 at a plurality of sheet joined portions 40 arranged at intervals as illustrated in Fig. 7, Fig. 9, etc. Further, a region having this elastic sheet stretchable structure includes a stretchable region that contracts in the width direction by contraction of the elastic sheet and is extensible in the width direction (that is, the stretchable direction ED is the width direction WD of the diaper).

A planar shape of the outer member 20 is formed by concave around-leg lines 29 so that both side edges of the intermediate portion L in the width direction form leg openings, respectively, and has a shape similar to an hourglass as a whole. The outer member 20 may be formed separately in the front body F and the back body B, and both bodies may be disposed to be separated in the front-back direction LD of the diaper at the crotch portion.

An embodiment illustrated in Fig. 1 and Fig. 2 is an embodiment in which a stretchable structure according to a conventional elongated waist portion elastic member 24 is provided without providing the elastic sheet stretchable structure 20X in waist end portions 23. However, it is possible to adopt an embodiment in which the elastic sheet stretchable structure 20X is extended to the waist end portions 23. The waist portion elastic members 24 are elongated elastic members such as a plurality of rubber threads disposed at intervals in the front-back direction LD, and apply a stretching force to tighten a waist of a body. The waist portion elastic members 24 are not disposed substantially in a bundle at close intervals, and three or more waist portion elastic members 24, preferably five or more waist portion elastic members 24 are disposed at intervals of about 3 to 8 mm in the front-back direction to form a predetermined stretchable zone. A stretch rate the waist portion elastic members 24 at the time of fixing can be determined as appropriate, and may be set to about 230 to 320% for a normal adult. As the waist portion elastic members 24, rubber threads are used in the illustrated example. However, other elongated elastic members such as flat rubber may be used. Although not illustrated, the elastic sheet 30 may be provided at the waist end portions 23, and the elongated waist portion elastic members 24 may be provided at positions overlapping the elastic sheet 30, so that a stretchable structure using both elastic members can be provided. In addition, in the illustrated embodiment, the stretchable region of the elastic sheet stretchable structure 20X is present up to edge portion regions 82 of leg openings in the outer member 20. For this reason, the elongated elastic members extending along leg openings are not provided in the edge portion regions 82 of the leg openings in the outer member 20. However, the elongated elastic members may be provided at positions overlapping the elastic sheet 30 in the edge portion regions 82 or instead of the elastic sheet 30 of the edge portion regions 82.

As other embodiments, although not illustrated, appropriate modifications can be made. For example, the elastic sheet stretchable structure 20X may not be provided in the intermediate portion L between the lower torso portion T of the front body F and the lower torso portion T of the back body B, the elastic sheet stretchable structure 20X may be continuously provided in the front-back direction LD from the inside of the lower torso portion T of the front body F to the inside of the lower torso portion T of the back body B via the intermediate portion L, or the elastic sheet stretchable structure 20X may be provided only in one of the front body F and the back body B.

### (Stretchable region)

A region having the elastic sheet stretchable structure 20X in the outer member 20 has a stretchable region that can be stretched and contracted in the width direction WD. The stretchable region 80 contracts in the width direction WD by a contraction force of the elastic sheet 30 and is extensible in the width direction WD. More specifically, in a state where the elastic sheet 30 is stretched in the width direction WD, the first sheet layer 20A and the second sheet layer 20B are bonded through the joint holes 31 of the elastic sheet 30 at intervals in each of the width direction WD and the front-back direction LD orthogonal thereto (the direction LD orthogonal to the stretchable direction)to form a plurality of sheet joined portions 40, thereby forming the elastic sheet stretchable structure 20X. Further, in the stretchable region 80, the elastic sheet 30 is left without disconnection in the width direction WD, and the sheet joined portions 40 are disposed such that the first sheet layer 20A and the second sheet layer 20B contract by the contraction force of the elastic sheet 30 and contraction pleats 25 are formed, thereby imparting such elasticity.

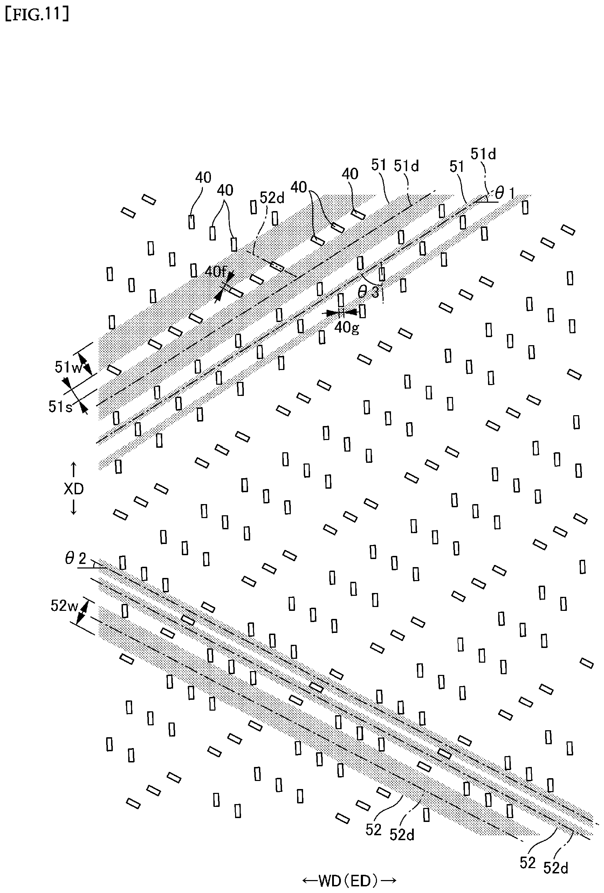

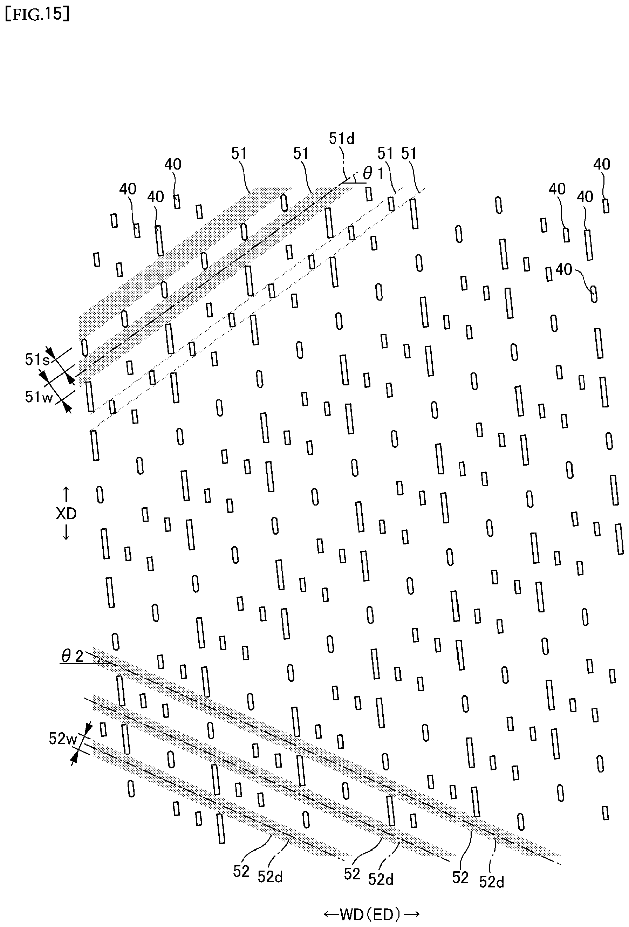

The stretchable region 80 may have a portion 32 in which the elastic sheet 30 is linearly continuous along the width direction WD as in an example illustrated in Fig. 7 and Fig. 9 or may not have the portion 32 as in an example illustrated in Fig. 11 and an example illustrated in Fig. 15.

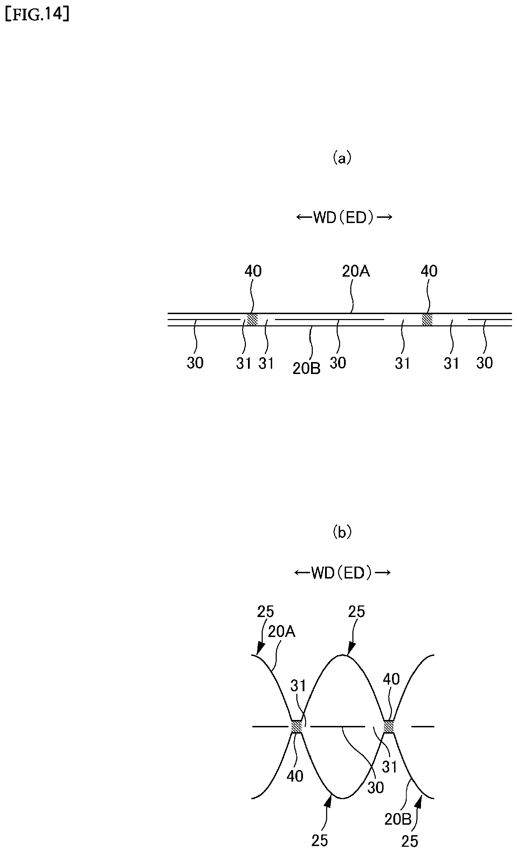

In the stretchable region, the first sheet layer 20A and the second sheet layer 20B between the sheet joined portions 40 swell in a direction in which they are separated from each other, thereby forming contraction pleats extending in the front-back direction LD in the natural length state as illustrated in Fig. 7(d), Fig. 9(d), and Fig. 14(b). Further, in the worn state of being stretched to some extent in the width direction WD, the contraction pleats 25 are left even though the contraction pleats 25 are extended. In addition, as in the illustrated embodiment, when the first sheet layer 20A and the second sheet layer 20B are not bonded to the elastic sheet 30 at least in a portion other than between the first sheet layer 20A and the second sheet layer 20B in the sheet joined portions 40, gaps are formed between the joint holes 31 and the sheet joined portions 40 in the elastic sheet 30, as can be seen from Fig. 7(c) and Fig. 9(c) assuming a worn state and Figs. 7(a) and 7(b) and Figs. 9(a) and 9(b) assuming an unfolded state of the first sheet layer 20A and the second sheet layer 20B, in these states. Even when the material of the elastic sheet 30 is a non-porous film or sheet, air permeability is imparted by the gaps. In particular, in the case where the elastic sheet 30 has the portion 32 which is linearly continuous along the width direction WD, the joint holes 31 narrow due to further contraction of the elastic sheet 30 and a gap is hardly formed between the joint holes 31 and the sheet joined portions 40 in the natural length state. When the elastic sheet 30 does not have the linearly continuous portion along the width direction WD, a gap remains between the joint holes 31 and the sheet joined portions 40.

It is desirable that a maximum elongation of the stretchable region 80 in the width direction WD is 190% or more (preferably 200 to 2200). The maximum elongation of the stretchable region 80 is substantially determined by the stretch rate of the elastic sheet 30 at the time of manufacture, and the maximum elongation decreases due to factors that inhibit contraction in the width direction WD based thereon. A main factor of such inhibition is a ratio of the length L of the sheet joined portions 40 per unit length in the width direction WD, and the maximum elongation decreases as this ratio increases. In a normal case, since the length L of the sheet joined portions 40 has a correlation with an area ratio of the sheet joined portions 40, the maximum elongation of the stretchable region 80 can be adjusted by the area ratio of the sheet joined portions 40.

As in the example illustrated in Fig. 7 and Fig. 9, in the case where the elastic sheet 30 has a portion 32 which is linearly continuous along the width direction WD, the stretching stress of the stretchable region 80 can be adjusted mainly by a sum of orthogonal dimensions 32w (equal to intervals 31d of the joint holes) of the portion 32 in which the elastic sheet 30 is linearly continuous along the width direction WD (see Fig. 7(a) and Fig. 9(a)). On the other hand, as in the example illustrated in Fig. 11 and the example illustrated in Fig. 15, in the case where the elastic sheet 30 has not the portion which is linearly continuous along the width direction WD, the stretching stress can be adjusted by an intersecting angle between the continuous direction of the non-joint bands 51 and 52 and the stretchable direction ED. In a normal case, it is preferable that each of the acute intersecting angles θ1 and θ2 between the continuous direction of the non-joint bands 51 and 52 and the stretchable direction ED in the unfolded state is set to be more than 0 degrees and 45 degrees or less, particularly a range of 10 to 30 degrees.

The area ratio of the sheet joined portions 40 and the area of each of the sheet joined portions 40 in the stretchable region 80 can be determined as appropriate and are preferably within the following ranges in a normal case.
Area of each of sheet joined portions 40: 0.14 to 3.5 mm² (particularly 0.14 to 1.0 mm²)
Area ratio of sheet joined portions 40: 1.8 to 19.1% (particularly 1.8 to 10.6%)

As described above, the maximum elongation and stretching stress of the stretchable region 80 can be adjusted by the area of the sheet joined portions 40. Thus, as illustrated in Fig. 1 and Fig. 2, a plurality of regions having different area ratios of the sheet joined portions 40 may be provided in the stretchable region 80 to change fitting according to the site. In the embodiment illustrated in Fig. 1 and Fig. 2, edge portion regions 82 of leg openings correspond to a flexibly stretching and contracting region in which the area ratio of the sheet joined portions 40 is high comparing to other regions, and thus the stretching stress is weak.

A shape of each of the sheet joined portions 40 and the joint holes 31 in the natural length state can be determined as appropriate, and may be set to any shape such as a perfect circle, an ellipse, a polygon such as a triangle, a rectangle, or a rhombus, a convex lens shape, a concave lens shape, a star shape, a cloud shape, etc. The dimensions of the individual sheet joined portions are not particularly limited. However, a maximum length 40y (approximately equal to a dimension 31y of the joint holes 31 in the orthogonal direction) is preferably 0.5 to 3.0 mm, particularly preferably 0.7 to 1.1 mm, and a maximum width 40x is preferably 0.1 to 3.0 mm, particularly 0.1 to 1.1 mm in the case of a shape that is long in a direction XD orthogonal to the stretchable direction.

A size of each of the sheet joined portions 40 may be determined as appropriate. When the size is excessively large, an influence of hardness of the sheet joined portions 40 on the feel increases. When the size is excessively small, a bonding area is small, and materials may not sufficiently adhere to each other. Thus, in a normal case, the area of each of the sheet joined portions 40 is preferably set to about 0.14 to 3.5 mm². It is sufficient that the area of an opening of each of the joint holes 31 is greater than or equal to the area of each of the sheet joined portions since the sheet joined portions are formed through the joint holes 31. However, the area is preferably about 1 to 1.5 times the area of each of the sheet joined portions. Incidentally, the area of the opening of each of the joint holes 31 refers to a value in a state of being integrated with the first sheet layer 20A and the second sheet layer 20B, not in a state of the elastic film 30 alone, and in the natural length state, and refers to a minimum value when the area of the opening of each of the joint holes 31 is not uniform in the thickness direction, for example, the area is different between the front and the back of the elastic film 30.

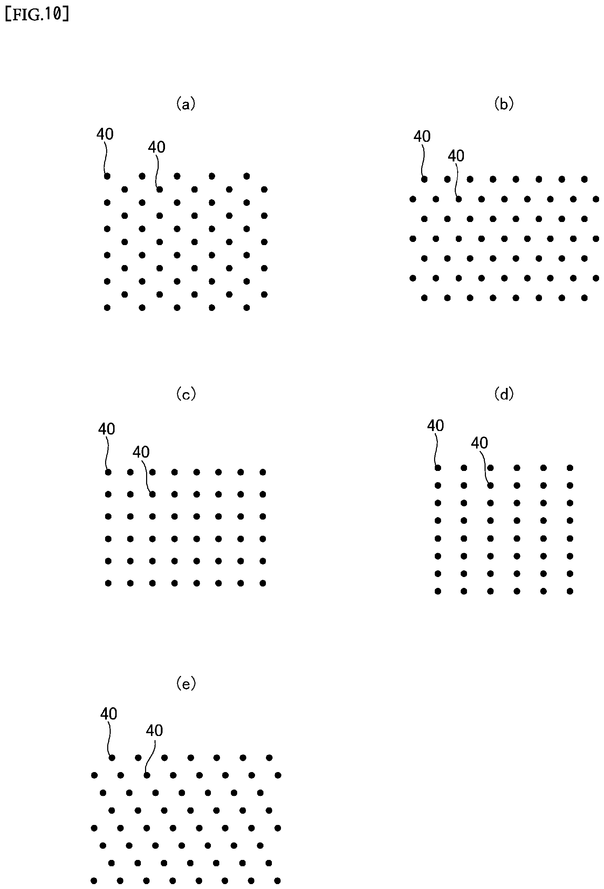

A planar arrangement of the sheet joined portions 40 and the joint holes 31 can be determined as appropriate. However, a regularly repeated planar arrangement is preferable. In addition to a regularly repeated planar arrangement such as an oblique lattice shape illustrated in Fig. 10(a), a hexagonal lattice shape illustrated in Fig. 10(b) (these shapes are also referred to as staggered shapes), a square lattice shape illustrated in Fig. 10(c), a rectangular lattice shape illustrated in Fig. 10(d), or a parallel lattice shape illustrated in Fig. 10(e) (a form in which two groups of a plurality of parallel rows in an oblique direction are provided to intersect each other as illustrated in the figure) (including shapes obtained by inclining these shapes at an angle less than 90 degrees with respect to the stretchable direction), it is possible to adopt an arrangement in which a group of the sheet joined portions 40 (arrangement in a group unit may be regular or irregular and may correspond to a pattern, a character shape, etc.) is regularly repeated.

An arrangement pattern of the sheet joined portions 40 in the stretchable region 80 is preferably as in the example illustrated in Fig. 9, as in the example illustrated in Fig. 11, and as in the example illustrated in Fig. 15. That is, in these examples, in the stretchable region 80, as non-joint bands 51 and 52 in which a part not having the sheet joined portions 40 is continuous in the unfolded state, a first non-joint band 51 linearly continuous along a first direction 51d intersecting the stretchable direction ED at an acute angle (acute angle θ1 formed by intersecting) is repeatedly present at intervals in a direction orthogonal to the first direction 51d. In addition, a plurality of sheet joined portions 40 and joint holes 31 are provided at intervals between adjacent first non-joint bands 51 in the stretchable region 80. Further, characteristically, a unit structure including a plurality of first non-joint bands 51 having different first widths 51w determined as widths in the direction orthogonal to the first direction 51d is repeatedly present in the direction orthogonal to the first direction 51d in the stretchable region 80.

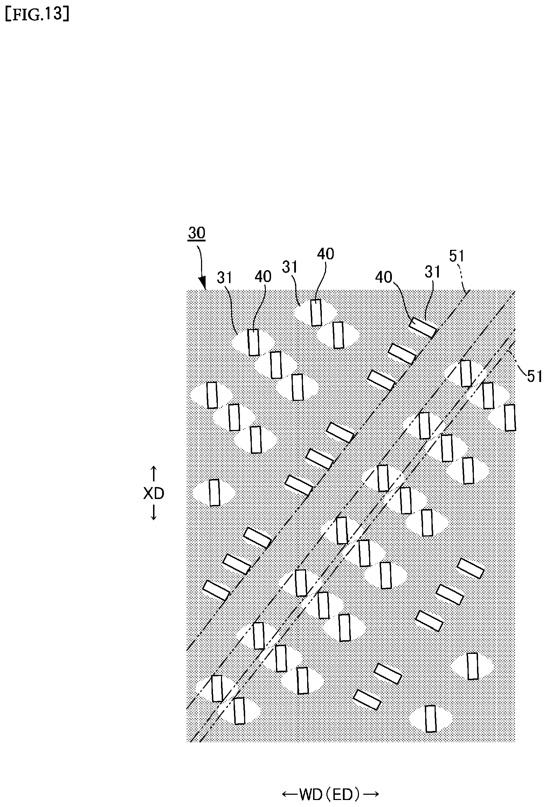

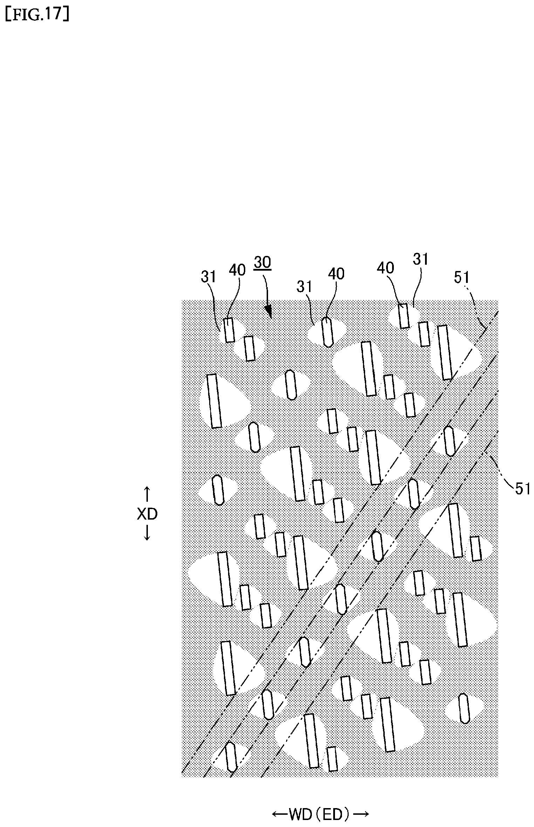

As described above, when the unit structure including the plurality of first non-joint bands 51 having different first widths 51w is repeatedly present in the direction orthogonal to the first direction 51d in the stretchable region 80, a similar magnitude change in width is formed in a continuous portion of the elastic sheet 30 inside the first non-joint bands 51. That is, when the width 51w of the first non-joint bands 51 is narrow, the width of the continuous portion of the elastic sheet 30 on the inside is narrowed. Further, when the width 51w of the first non-joint bands 51 is wide, the width of the continuous portion of the elastic sheet 30 on the inside is widened. Further, when there is a change in the first width 51w in the continuous portion of the elastic sheet 30 in the first non-joint bands 51, both the continuous portion of the elastic sheet 30 in first non-joint bands 51 having a wide width and the continuous portion of the elastic sheet 30 in first non-joint bands 51 having a narrow width are visually emphasized. As a result, regardless of whether the stretchable region 80 is in the natural length state (see Fig. 13 and Fig. 17) or in the worn state stretched to some extent, an appearance having beautiful oblique stripe patterns is exhibited. In addition, in a state of being contracted to some extent, a size of the contraction pleats 25 in the first non-joint bands 51 changes according to the first width 51w of the first non-joint bands 51, and thus an oblique stripe pattern appears more clearly due to an influence of the contraction pleats 25.

The unit structure described above is not limited by the magnitude of the width 51w as long as the plurality of first non-joint bands 51 having different first widths 51w is included. However, it is preferable that a large first width 51w in the first non-joint bands 51 is 1.2 to 60 times that of a first non-joint band 51 having a closest width 51w and a small first width 51w is 0.01 to 0.8 times that of the first non-joint band 51 having the closest width 51w.

In addition, in the unit structure described above, as long as the plurality of first non-joint bands 51 having the different first widths 51w is included, the first widths 51w in all the first non-joint bands 51 may be different from each other, and a first width 51w in some of the plurality of non-joint bands 51 may be different from a first width 51w of one or a plurality of other first non-joint bands 51 as illustrated in the figure.

Even if an oblique stripe pattern along the first direction 51d due to the contraction pleats 25 of the first non-joint bands 51 and the continuous portion of the elastic sheet 30 therein appears in the stretchable region 80, when an oblique stripe pattern along another oblique direction is more strongly visually recognized in the same stretchable region 80, there is concern that the oblique stripe pattern due to the contraction pleats 25 of the first non-joint bands 51 and the continuous portion of the elastic sheet 30 therein becomes inconspicuous. On the other hand, it is preferable that a maximum value of the first widths 51w in the first non-joint bands 51 is a maximum value of widths in a direction orthogonal to a continuous direction in all the common non-joint bands 51 and 52 having different inclination directions since an oblique stripe pattern due to the contraction pleats 25 of the first non-joint bands 51 and the continuous portion of the elastic sheet 30 therein is more strongly visually recognized in the stretchable region 80. In this case, the maximum value of the first widths 51w in the first non-joint bands 51 can be determined as appropriate, and is preferably 0.01 to 9 times that of the first non-joint band 51 having the closest width 51w. Incidentally, widths of all the non-joint bands 51 and 52 including the first non-joint bands 51 in the direction orthogonal to the continuous direction are not limited and are preferably within a range of 0.3 to 50 mm in a normal case. Naturally, the widths of the non-joint bands 51 and 52 in the direction orthogonal to the continuous direction are the first width 51w in the first non-joint bands 51, which correspond to the linearly continuous portion. Therefore, the widths are equal widths.

A first interval 51s determined as an interval between the adjacent first non-joint bands 51 in the direction orthogonal to the first direction 51d can be determined as appropriate. Therefore, the first interval 51s may be the same as, wider than, or narrower than the first width 51w of the adjacent first non-joint bands 51. As one preferable example, it is possible to mention a mode in which the maximum value of the first widths 51w of the first non-joint bands 51 is smaller than a maximum value of the first interval 51s in the unit structure. In this way, by forming a wide interval portion in the unit structure, the oblique stripe pattern due to the contraction pleats 25 of the first non-joint bands 51 and the continuous portion of the elastic sheet 30 inside thereof is more strongly visually recognized. In this case, the maximum value of the first widths 51w of the first non-joint bands 51 can be determined as appropriate, and is preferably 0.01 to 9 times the maximum value of the first interval 51s. Incidentally, intervals between all the non-joint bands 51 and 52 including the first non-joint bands 51 in the direction orthogonal to the continuous direction are not particularly limited and are preferably within a range of 0.3 to 50 mm in a normal case. Naturally, the intervals between the non-joint bands 51 and 52 in the direction orthogonal to the continuous direction correspond to the first interval 51s in the first non-joint bands 51 and correspond to equal intervals in the continuous direction.

In the stretchable region 80, as non-joint bands 51 and 52, the second non-joint bands 52 linearly continuous along a second direction 52d intersecting the stretchable direction ED at an acute angle (acute intersecting angle θ2) other than the first direction 51d may be repeatedly present at intervals in a direction orthogonal to the second direction 52d, or the second non-joint bands 52 may not be present. In one preferable mode having the second non-joint bands 52, the non-joint bands 51 and 52 are formed in an oblique lattice shape in the stretchable region 80, the first non-joint bands 51 are continuous portions in one direction in the non-joint bands 51 and 52 having the oblique lattice shape, and the second non-joint bands 52 are continuous portions in another direction in the non-joint bands 51 and 52 having the oblique lattice shape. In this case, the first direction 51d and the second direction 52d are opposite to each other in terms of inclination with respect to the stretchable direction ED. Incidentally, as in the example illustrated in Fig. 11 and the example illustrated in Fig. 15, even in a mode not having the non-joint bands 51 and 52 continuous in the width direction WD (stretchable direction ED), when each of the acute intersecting angles θ1 and θ2 between the first and second directions 51d and 52d and the stretchable direction ED is 5 to 45 degrees, particularly 10 to 30 degrees in the unfolded state of the stretchable region 80, elasticity in the stretchable region 80 can be sufficiently ensured.

However, when an oblique stripe pattern along an oblique direction of the second non-joint bands 52 is more strongly visually recognized in the same stretchable region 80, there is concern that the oblique stripe pattern due to the contraction pleats 25 of the first non-joint bands 51 and the continuous portion of the elastic sheet 30 therein becomes inconspicuous. Therefore, in the case where the second non-joint bands 52 is present as in the example illustrated in Fig. 15, it is desirable that all the second widths 52w determined as a width in the direction orthogonal to the second direction in the second non-joint bands 52 are the same, or the sheet joined portions 40 are disposed so that the second non-joint bands 52 is not present. In this way, in the stretchable region 80, the oblique stripe pattern due to the contraction pleats 25 of the first non-joint bands 51 and the continuous portion of the elastic sheet 30 inside thereof is more strongly visually recognized.

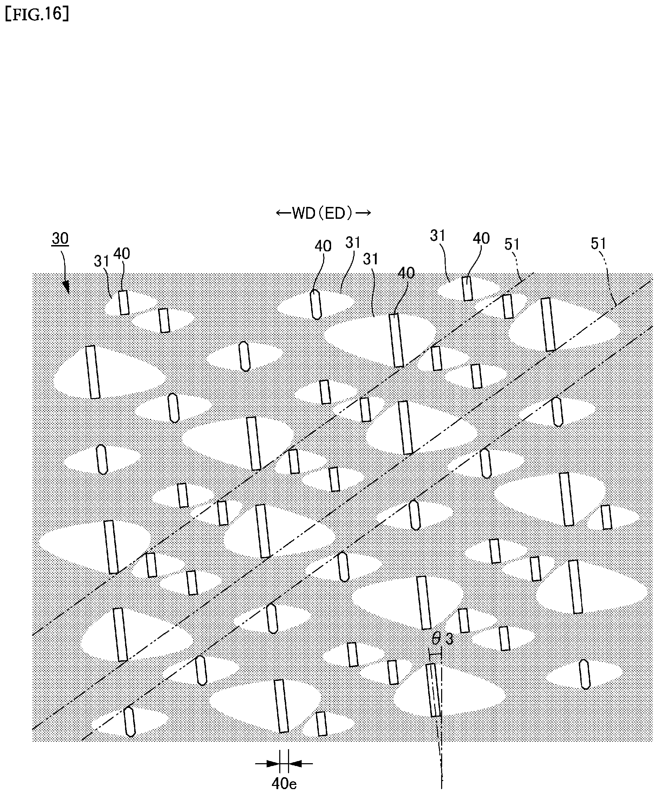

Meanwhile, between adjacent first non-joint bands 51, the sheet joined portions 40 are aligned in the first direction 51d. In this case, for example, as illustrated in Fig. 16, it is preferable that all the sheet joined portions 40 have an elongated shape in which an acute angle θ3 formed by intersecting of the longitudinal direction with respect to the direction orthogonal to the stretchable direction ED is within 10 degrees and a maximum dimension 40e in the stretchable direction ED is 0.1 to 0.4 mm since it is possible to ensure a larger dimension of the first non-joint bands 51 in the stretchable direction ED and to suppress a decrease in elasticity.

In addition, as in the example illustrated in Fig. 11, when the unit structure includes a plurality of first wide non-joint bands 51 having a maximum first width 51w and a plurality of first narrow non-joint bands 51 having a narrower first width 51w adjacent to each other in the direction orthogonal to the first direction 51d, it is preferable that sheet joined portions 40 having an elongated shape in which the acute angle formed by intersecting of the longitudinal direction with respect to the second direction 52d is within 5 degrees and a maximum dimension 40f in the direction orthogonal to the longitudinal direction is 0.1 to 0.4 mm are aligned at intervals in the first direction 51d between the adjacent first wide non-joint bands 51. In addition, it is preferable that sheet joined portions 40 having an elongated shape in which the acute angle θ3 formed by intersecting of the longitudinal direction with respect to the first direction 51d is 45 degrees or more and a maximum dimension 40g in the direction orthogonal to the longitudinal direction is 0.1 to 0.4 mm are aligned at intervals in the first direction 51d between the adjacent first narrow non-joint bands 51. By such a shape and arrangement of the sheet joined portions 40, the contraction pleats 25 of the first non-joint bands 51 and the continuous portion of the elastic sheet 30 therein are particularly visually emphasized due to the small area of sheet joined portions 40.

One row or a plurality of rows of the sheet joined portions 40 (rows of the non-joint bands 51 and 52 in the continuous direction) may be located between the adjacent non-joint bands 51 and 52. In addition, it is preferable that intervals between the sheet joined portions 40 in a row direction are regular. However, all the intervals may not be constant, and some intervals may be different.

### (Non-stretchable region)

In a region having the elastic sheet stretchable structure 20X in the outer member 20, as illustrated in Fig. 2, a non-stretchable region 70 may be provided at least on one side of the stretchable region 80 in the width direction. The non-stretchable region 70 means that a maximum elongation in the stretchable direction is 120% or less. The maximum elongation of the non-stretchable region 70 is preferably 110% or less, and more preferably 100%. Arrangement of the stretchable region 80 and the non-stretchable region 70 can be determined as appropriate. In the case of the outer member 20 of the underpants-type disposable diaper as in the present example, a portion overlapping the absorbent body 13 is a region not requiring stretching and contraction. Thus, as in the illustrated embodiment, it is preferable to form a part or all of the portion overlapping the absorbent body 13 (it is desirable to include almost the entire inner/outer fixing region 10B) into the non-stretchable region 70. Naturally, the non-stretchable region 70 may be provided from a region overlapping the absorbent body 13 to a region not overlapping the absorbent body 13 away from the region in the width direction WD or the front-back direction LD, and the non-stretchable region 70 may be provided only in the region not overlapping the absorbent body 13.

The shape of each of the sheet joined portions 40 in the non-stretchable region 70 is not particularly limited, and may be appropriately selected from the same shapes as those described in the section of the stretchable region 80.

In addition, the area ratio of the sheet joined portions 40 and the area of each of the sheet joined portions 40 in the non-stretchable region 70 can be determined as appropriate. However, in a normal case, the area ratio and the area are preferably within the following ranges since the non-stretchable region 70 does not become hard due to the small area of each of the sheet joined portions 40 and the low area ratio of the sheet joined portions 40.
Area of each of sheet joined portions 40: 0.10 to 0.75 mm² (particularly 0.10 to 0.35 mm²)
Area ratio of sheet joined portions 40: 4 to 13% (particularly 5 to 10%)

The non-stretchable region 70 can be formed by densely disposing the sheet joined portions 40 so that the first sheet layer and the second sheet layer are prevented from being contracted by the contraction force of the elastic sheet 30 to form pleats. Specific examples of a method for forming the non-stretchable region 70 include those shown in, for example, JP 5980355 B2, JP 5918877 B2, JP 5980367 B2, and JP 6049228 B2.

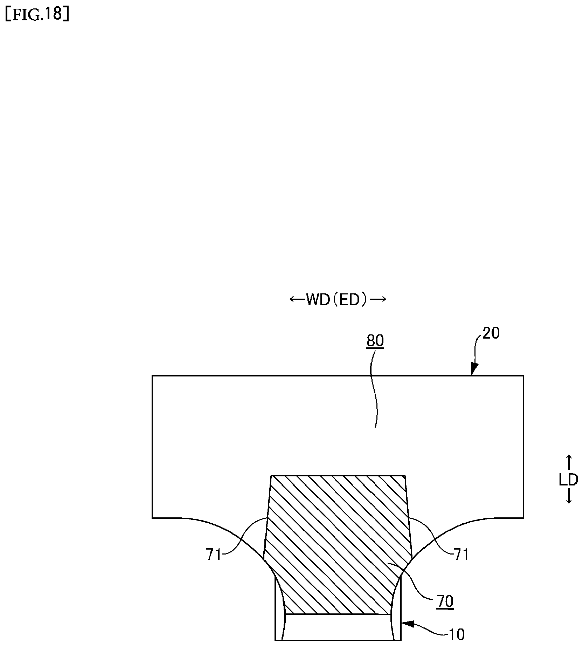

By changing the area ratio of the sheet joined portions 40, it is possible to change an elasticity property. However, when a position at which the area ratio of the sheet joined portions 40 rapidly changes, that is, a boundary between the stretchable region 80 and the non-stretchable region 70 extends along the orthogonal direction XD orthogonal to the stretchable direction ED from one end to the other end thereof, a linear pressure rapidly changes at the boundary between the stretchable region 80 and the non-stretchable region 70 during welding. Such a rapid change in linear pressure may lead to early wear or breakage of equipment such as an anvil roll. Therefore, when the area ratio of the sheet joined portions 40 in the stretchable region 80 is 0.5 to 1 times the area ratio of the sheet joined portions 40 in the non-stretchable region 70, it is preferable that the boundary 71 between the stretchable region 80 and the non-stretchable region 70 has a shape continuously shifted only to one side in the stretchable direction ED from one end to the other end thereof as illustrated in Fig. 2 and Fig. 18 to Fig. 20. In this way, there is no rapid change in linear pressure during welding, and thus there is little concern about leading to early wear or breakage of equipment such as an anvil roll.

However, in the underpants-type disposable wearing article, when the boundary 71 between the stretchable region 80 and the non-stretchable region 70 has a shape continuously shifted only to a center side in the width direction WD from an end on the waist opening side to a center of the disposable wearing article in the front-back direction LD, the stretchable region 80 on the waist opening side shortens, which is not preferable from a viewpoint of ensuring fitting. Therefore, as in the illustrated example, it is preferable that a position of the boundary 71 between the stretchable region 80 and the non-stretchable region 70 is continuously shifted only to sides of the side seal portions 21 from the end on the waist opening side to the center of the disposable wearing article in the front-back direction LD.

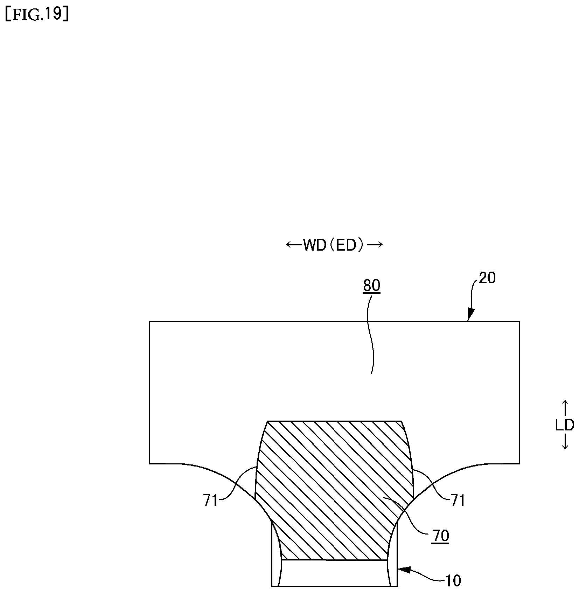

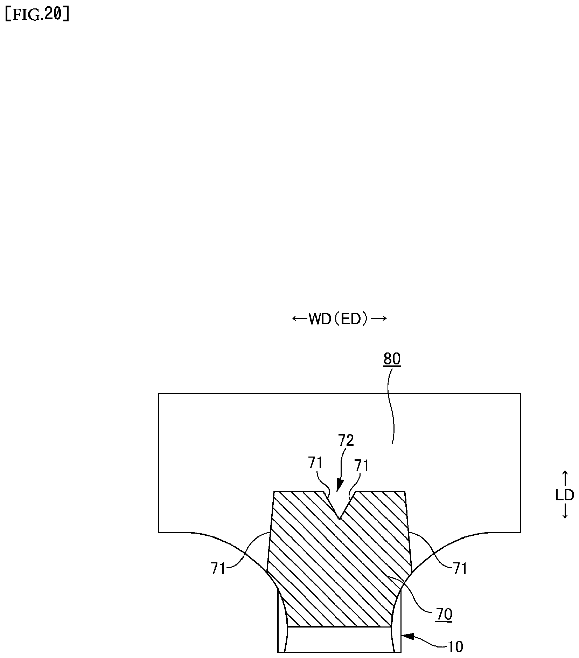

The boundary 71 between the stretchable region 80 and the non-stretchable region 70 may have a linear shape as examples illustrated in Fig. 2, Fig. 18, and Fig. 20 or a curved shape as an example illustrated in Fig. 19. When a concave portion 72 recessed to a crotch side is formed in a portion on the waist opening side of the non-stretchable region 70 and the concave portion 72 is used as the stretchable region 80 as in the example illustrated in Fig. 20, the boundary 71 between the stretchable region 80 and the non-stretchable region 70 is generated on both sides of the concave portion 72 in the stretchable direction ED. Therefore, it is desirable that the boundary 71 between the stretchable region 80 and the non-stretchable region 70 is similarly inclined in this portion.

### (Bonding structure of sheet joined portions)

When the first sheet layer 20A and the second sheet layer 20B are bonded in the sheet joined portions 40 through the joint holes 31 formed in the elastic sheet 30, it is desirable that the first sheet layer 20A and the second sheet layer 20B are not bonded to the elastic sheet 30 except at least between the first sheet layer 20A and the second sheet layer 20B in the sheet joined portions 40.

When the first sheet layer 20A and the second sheet layer 20B are welded through the joint holes 31 of the elastic sheet 30 at the sheet joined portions 40, both the first sheet layer 20A and the second sheet layer 20B may be melted and solidified at the sheet joined portions 40, or only one of the first sheet layer 20A and the second sheet layer 20B may be melted and solidified at the sheet joined portions 40. Further, a molten and solidified material of the elastic sheet 30 may be interposed in the sheet joined portions 40.

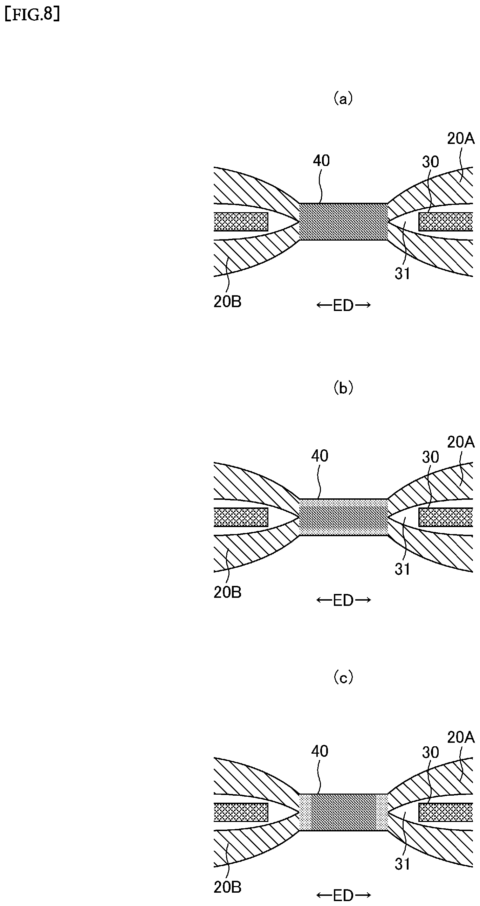

The first sheet layer 20A and the second sheet layer 20B may be uniformly melted and solidified throughout the sheet joined portions 40 in a thickness direction and a planar direction as in the example illustrated in Fig. 8(a) or non-uniformly melted and solidified as indicated by dot pattern gradation in Figs. 8(b) and 8(c). For example, the first sheet layer 20A and the second sheet layer 20B may have a lower degree of melting toward the outside in the thickness direction of the sheet joined portions 40 as in the example illustrated in Fig. 8(b). This state includes a state in which almost all fibers of the first sheet layer 20A and the second sheet layer 20B are not melted on the surface of the sheet joined portions 40, a state in which molten and solidified materials and unmelted fibers of the first sheet layer 20A and the second sheet layer 20B are mixed on the surface of the sheet joined portions 40, and a state in which fibers of the first sheet layer 20A and the second sheet layer 20B are melted throughout the sheet joined portions 40 in the thickness direction and a degree of melting changes.

With or without a change in the degree of melting in the thickness direction in the sheet joined portions 40, the degree of melting of the first sheet layer 20A and the second sheet layer 20B may be lower toward the peripheral side of the sheet joined portions 40 as in the example illustrated in Fig. 8(c). This state includes a state in which almost all fibers of the first sheet layer 20A and the second sheet layer 20B are not melted at peripheral edges of the sheet joined portions 40 (however, only when the molten and solidified material of the elastic sheet 30 described above is interposed as an adhesive), a state in which molten and solidified materials and unmelted fibers of the first sheet layer 20A and the second sheet layer 20B are mixed at the peripheral edges of the sheet joined portions 40, and a state in which fibers of the first sheet layer 20A and the second sheet layer 20B are melted throughout the sheet joined portions 40 in the planar direction and a degree of melting changes.

Incidentally, in these states, the fact that the fibers of the first sheet layer 20A and the second sheet layer 20B are melted includes the fact that cores of the fibers (not only a core in a composite fiber but also a center portion of a single component fiber) are left and surrounding parts thereof (including not only a sheath in the composite fiber but also a part of the single component fiber on the surface layer side) are melted in addition to the fact that all the fibers are melted.

In addition, a state in which the molten and solidified material of the elastic sheet 30 is left in the sheet joined portions 40 includes a state of being left in a layer shape while being hardly mixed with the first sheet layer 20A or the molten and solidified material and the second sheet layer 20B or the molten and solidified material thereof therebetween, a state of being mixed with melted and solidified one of the first sheet layer 20A and the second sheet layer 20B, and a state of penetrating to some extent between fibers of not melted and solidified one of the first sheet layer 20A and the second sheet layer 20B or between remaining fibers (including cores) in melted and solidified one of the first sheet layer 20A and the second sheet layer 20B.

In the state in which the molten and solidified material of the elastic sheet 30 is left in the sheet joined portions 40, under the condition that a melting point of at least one of the first sheet layer 20A and the second sheet layer 20B is higher than a melting point of the elastic sheet 30, the elastic sheet 30 is interposed between the first sheet layer 20A and the second sheet layer 20B, a site corresponding to the sheet joined portions 40 is pressurized and heated, and at least one of the first sheet layer 20A and the second sheet layer 20B and the elastic sheet 30 are melted. In this way, manufacturing can be performed.

In this case, the melting point of the elastic sheet 30 is preferably about 80 to 145°C, the melting points of the first sheet layer 20A and the second sheet layer 20B are preferably about 85 to 190°C, particularly 150 to 190°C, and a difference between the melting point of the first sheet layer 20A and the second sheet layer 20B and the melting point of the elastic sheet 30 is preferably about 60 to 90°C. In addition, the heating temperature is preferably set to about 100 to 150°C.

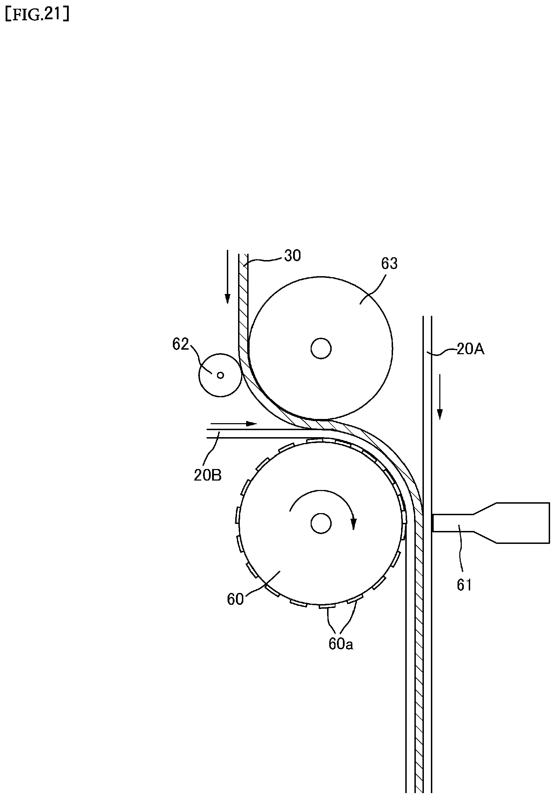

Fig. 21 illustrates an example of a suitable ultrasonic sealing device. In this ultrasonic sealing device, when the sheet joined portions 40 are formed, the first sheet layer 20A, the elastic sheet 30, and the second sheet layer 20B are fed between an anvil roll 60 having projections 60a formed in the pattern of the sheet joined portions 40 on an outer surface and an ultrasonic horn 61. At this time, for example, by setting a feeding speed of the upstream elastic sheet 30 by a feed drive roll 63 and a nip roll 62 to be lower than a feeding speed on the downstream side of the anvil roll 60 and the ultrasonic horn 61, the elastic sheet 30 is stretched to a predetermined stretch rate in an MD (machine direction, flow direction) through a path from a nip position by the feed drive roll 63 and the nip roll 62 to a seal position by the anvil roll 60 and the ultrasonic horn 61. The stretch rate of the elastic sheet 30 can be set by selecting a speed difference between the anvil roll 60 and the feed drive roll 63, and can be set to about 300% to 500%, for example. Reference character 62 indicates the nip roll.

The first sheet layer 20A, the elastic sheet 30, and the second sheet layer 20B fed between the anvil roll 60 and the ultrasonic horn 61 are heated by ultrasonic vibration energy of the ultrasonic horn 61 while being pressurized between the projections 60a and the ultrasonic horn 61 in a state of being stacked in this order. By melting only the elastic sheet 30 or melting at least one of the first sheet layer 20A and the second sheet layer 20B and the elastic sheet 30, the joint holes 31 are formed in the elastic sheet 30. At the same time, the first sheet layer 20A and the second sheet layer 20B are bonded through the joint holes 31. Therefore, in this case, by selecting a size, a shape, a separation interval, and an arrangement pattern in a roll length direction and a roll circumferential direction of the projections 60a of the anvil roll 60, it is possible to select an area ratio of the sheet joined portions 40.

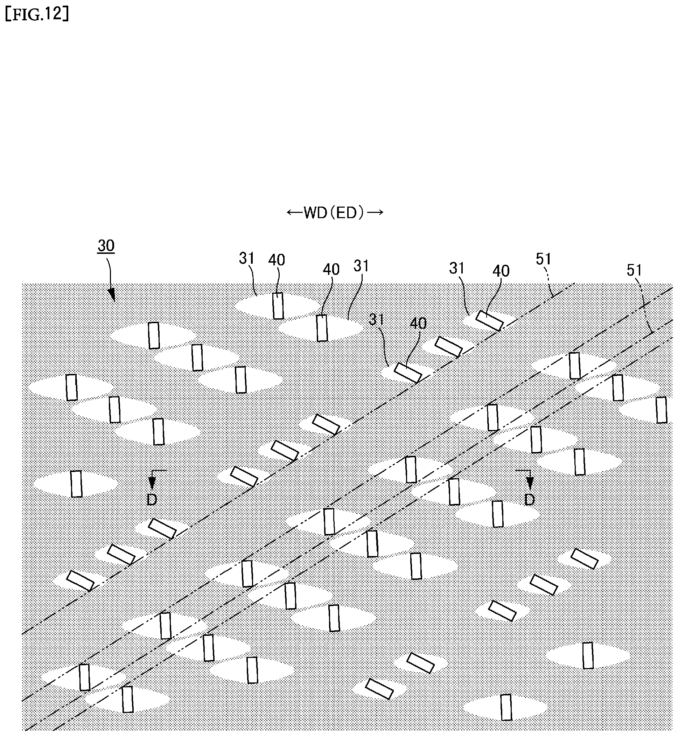

A reason why the joint holes 31 are formed may not be clear. However, it is considered that the holes are formed when portions corresponding to the projections 60a of the anvil roll 60 in the elastic sheet 30 are melted and detached from the surroundings. In this instance, a portion between adjacent joint holes 31 aligned in the stretchable direction ED in the elastic sheet 30 is cut from portions on both sides in the stretchable direction by the joint holes 31 as illustrated in Figs. 7(a) and 7(b), Figs. 9(a) and 9(b), Fig. 12, and Fig. 13, and loses support on both sides in a contracting direction. Thus, in a range in which continuity in a direction orthogonal to the contracting direction can be maintained, a center side in the direction LD orthogonal to the stretchable direction ED more contracts until the center side is balanced with a center side in the stretchable direction, and the joint holes 31 enlarge in the stretchable direction ED.

The constituent material of the first sheet layer 20A and the second sheet layer 20B can be used without particular limitation as long as at least a part of the fibers can be welded nonwoven fabric (that is, includes a thermoplastic resin component). For example, examples thereof may include a polyolefin-based synthetic fiber such as polyethylene or polypropylene, a polyester-based synthetic fiber, a polyamide-based synthetic fiber, etc., a mixed fiber in which two or more of these types are used, or a composite fiber containing two or more of these components (for example, a core-sheath type in which a sheath component is easily melt). Further, the nonwoven fabric may be manufactured by any processing.

As a method of fiber bonding in the nonwoven fabric, it is possible to adopt any one of chemical means such as an adhesive or a solvent, physical means such as heating, or so-called entanglement. For example, it is possible to adopt a spunlace method, a spunbond method, a thermal bond method, a meltblown method, a needle punch method, an air through method, a point bond method, etc. In the case of using a nonwoven fabric, a basis weight is preferably set to about 10 to 25 g/m². Further, a part or all of the first sheet layer 20A and the second sheet layer 20B may correspond to a pair of layers faced to each other by folding a single material. For example, as in the illustrated embodiment, in the waist end portions 23, a constituent material located on the outside may be used as the second sheet layer 20B, a folded portion 20C folded back to an internal surface side at a waist opening edge thereof may be used as the first sheet layer 20A, and the elastic sheet 30 may be interposed therebetween. Further, in other portions, a constituent material located on the inside may be used as the first sheet layer 20A, a constituent material located on the outside may be used as the second sheet layer 20B, and the elastic sheet 30 may be interposed therebetween. Naturally, the constituent material of the first sheet layer 20A and the constituent material of the second sheet layer 20B may be individually provided over the entire region in the front-back direction LD, and the elastic sheet 30 may be interposed between the constituent material of the first sheet layer 20A and the constituent material of the second sheet layer 20B without folding back the constituent materials.

The elastic sheet 30 is not particularly limited, and may correspond to a stretchable nonwoven fabric in addition to the elastic film as long as the sheet is made of a thermoplastic resin having elasticity. Further, as the elastic sheet 30, in addition to a non-porous sheet, it is possible to use a sheet in which a plurality of holes or slits is formed for ventilation. In particular, it is preferable that the elastic sheet 30 has a tensile strength in the width direction WD (stretchable direction ED, MD) of 8 to 25 N/35 mm, a tensile strength in the front-back direction LD (direction XD orthogonal to the stretchable direction, CD (cross direction)) of 5 to 20 N/35 mm, a tensile elongation in the width direction WD of 450 to 1,050%, and a tensile elongation in the front-back direction LD of 450 to 1,400%. A thickness of the elastic sheet 30 is not particularly limited. However, the thickness is preferably about 20 to 40 µm.

### (Combination of sheet joined portions having different shapes)

In a region having the elastic sheet stretchable structure 20X, the sheet joined portions 40 having different shapes may be provided to change the elasticity property or the appearance. For example, the sheet joined portions 40 in the stretchable region 80 of the example illustrated in Fig. 11 include rectangular sheet joined portions 40 having different orientations, and such sheet joined portions 40 having different orientations are included in the sheet joined portions 40 having different shapes. In addition, the sheet joined portions 40 in the stretchable region 80 of the example illustrated in Fig. 15 include rectangular sheet joined portions 40 having different lengths, and such sheet joined portions 40 having different lengths are included in the sheet joined portions 40 having different shapes. Further, a case in which the shapes of the sheet joined portions 40 in the stretchable region 80 are different from the shapes of the sheet joined portions 40 in the non-stretchable region 70 is naturally included in a case in which the region having the elastic sheet stretchable structure 20X includes the sheet joined portions 40 having different shapes.

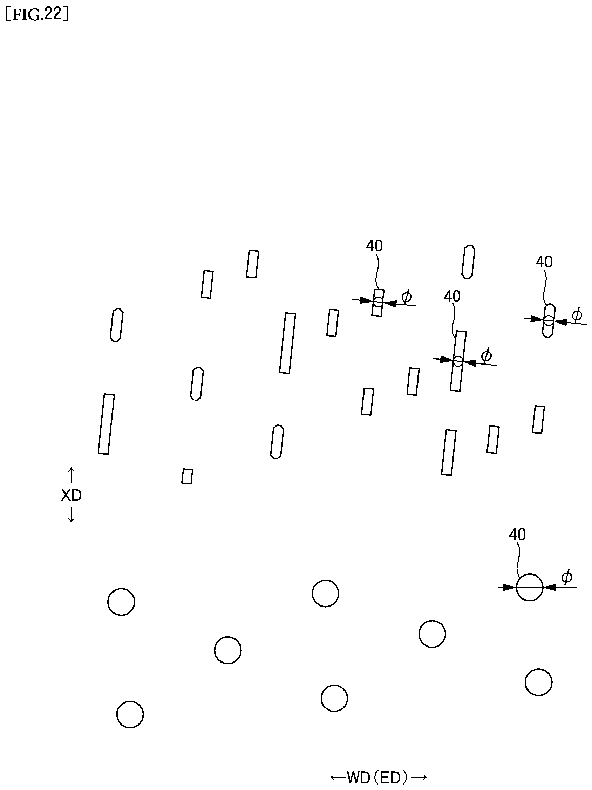

In the case of the underpants-type disposable wearing article illustrated in the example, it is preferable that the elastic sheet stretchable structure 20X is provided from the inside of the intermediate portion L to the inside of the lower torso portion T in the front-back direction LD and between the side seal portions 21 in the width direction WD, and the elasticity and appearance of the edge portion regions 82 along the leg openings are made different from the elasticity and appearance of other regions, thereby improving fitting of around-leg portions and other regions. To this end, for example, as enlarged and illustrated in Fig. 22, the shape of the sheet joined portions 40 in the edge portion regions 82 along the leg openings may be made different from the shape of the sheet joined portions 40 in other regions.

As described above, when the sheet joined portions 40 having different shapes are provided in the region having the elastic sheet stretchable structure 20X, there is concern that the a welding defect may occur, or there is concern about leading to early wear or breakage of equipment such as an anvil roll as described above. Therefore, in all the sheet joined portions 40 in the region having the elastic sheet stretchable structure 20X, a reference bonding diameter ϕ is 0.25 to 0.5 mm, and a circumference length of the sheet joined portions 40 is 1 to 15 times a length of a circumference of a circle whose diameter is the reference bonding diameter ϕ. The meaning of each numerical value is as described above. That is, when the reference bonding diameter is less than 0.25 mm, basically, a welding defect is likely to occur. Further, the sheet joined portions 40 having an excessively long or excessively complicated outer shape tend to cause a partial welding defect. From this viewpoint, the circumference length of the sheet joined portions 40 is within the above range. Further, in a case in which a difference in the reference bonding diameter ϕ between the sheet joined portions 40 is excessively large, when the linear pressure at the time of forming the sheet joined portions 40 is adjusted to that of a sheet joined portion 40 having a large reference bonding diameter ϕ, a welding defect is likely to occur. In addition, when the linear pressure at the time of forming the sheet joined portions 40 is adjusted to that of a sheet joined portion 40 having a small reference bonding diameter ϕ, there is concern about leading to early wear or breakage of equipment such as an anvil roll.

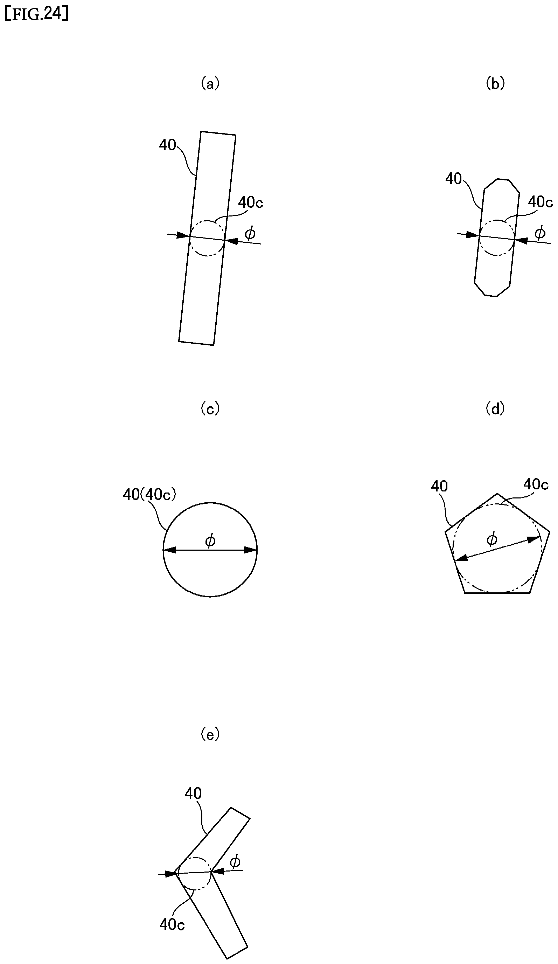

Here, the reference bonding diameter ϕ refers to a diameter of a largest inscribed circle 40c inscribed in the outer shape of the sheet joined portion 40. Therefore, a reference bonding diameter ϕ of an elongated sheet joined portion 40 is acute than a long diameter as illustrated in Figs. 24(a) and 24(b), and a reference bonding diameter ϕ of a sheet joined portion 40 having a shape of a circle or a shape similar to a circle such as a pentagon is almost the same as a long diameter as illustrated in Figs. 24(c) and 24(d). As illustrated in Fig. 24(e), in the case of an L-shaped sheet joined portion 40, a reference bonding diameter ϕ is smaller than a long diameter. As can be seen from these examples, although not illustrated, a sheet joined portion 40 having a complicated shape such as a star shape has a small reference bonding diameter ϕ.

It is sufficient that the size of the reference bonding diameter ϕ is within the above range. In the invention of this application, the first sheet layer and the second sheet layer are nonwoven fabrics having a fineness of 0.7 to 6 dtex and a basis weight of 10 to 25 g/m². In this way, it is possible to achieve both suppression of a welding defect and prevention of early wear/breakage of the equipment.

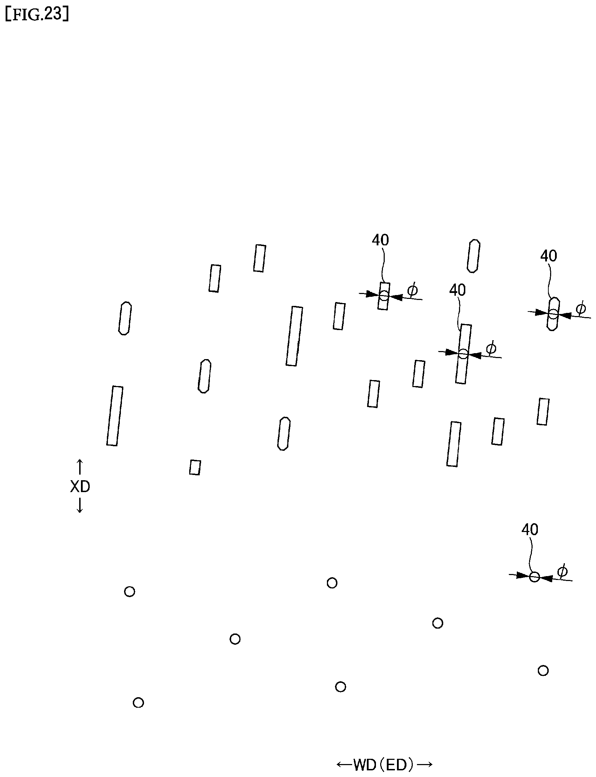

In the sheet joined portions 40 having different shapes, the reference bonding diameter ϕ may be different as in an example illustrated in Fig. 22 or the same as illustrated in Fig. 23.

The linear pressure that affects the welding defect in the ultrasonic seal can be adjusted by a force pressure. Thus, to surely prevent the welding defect, it is desirable that all the sheet joined portions 40 formed by one ultrasonic horn satisfy the above conditions. Similarly, in the case of heat sealing, it is desirable that all the sheet joined portions 40 formed by a pair of anvil rolls and opposing rolls satisfy the above conditions.

### <Description of terms in specification>

The following terms in the specification have the following meanings unless otherwise specified in the specification.
- The "front body" and the "back body" refer to portions on the front side and the back side, respectively, with respect to a center of the underpants-type disposable diaper in the front-back direction as a boundary. In addition, the crotch portion refers to a range in the front-back direction including the center of the underpants-type disposable diaper in the front-back direction, and refers to a range of a portion having a narrowing portion in the front-back direction when the absorbent body has the narrowing portion.
- The "maximum elongation" refers to a maximum value of an elongation in the stretchable direction ED (in other words, an elongation in the unfolded state in which the first sheet layer and the second sheet layer are unfolded flat without contraction or slack), and represents a length in the unfolded state as a percentage when the natural length is 100%.
- The "area ratio" refers to a ratio of a target portion to a unit area, and is represented as a percentage by dividing a total area of target portions (for example, the sheet joined portions 40, the openings of the joint holes 31, and the vent holes) in target regions (for example, the stretchable region 80 and the non-stretchable region 70) by an area of the target regions. In particular, the "area ratio" in a region having the stretchable structure refers to an area ratio in the unfolded state. In a mode in which a plurality of target portions is provided at intervals, it is desirable to obtain the area ratio by setting a size of the target regions to include ten or more target portions.
- The "stretch rate" refers to a value when the natural length is 100%.
- The "basis weight" is measured as below. A sample or a test piece is pre-dried, and then is left in a test room or a device in a standard state (temperature 23 ± 1°C, relative humidity 50 ± 2% in a test location), and is put in a constant weight state. Pre-drying refers to setting the weight of the sample or the test piece to a constant weight in an environment in which temperature is 100°C Incidentally, pre-drying is unnecessary for a fiber having an official moisture regain of 0.0%. A sample having dimensions of 100 mm × 100 mm is cut off from the test piece in the constant weight state using a sampling template (100 mm × 100 mm). A weight of the sample is measured and multiplied by 100 to calculate a weight per square meter, and the weight is set to the basis weight.
- The "thickness" of the absorbent body is measured using a thickness measuring instrument of Ozaki Mfg. Co., Ltd. (Peacock, Dial Thickness Gauge Large Type, Model J-B (measurement range 0 to 35 mm) or Model K-4 (measurement range 0 to 50mm)) by horizontally placing the sample and the thickness measuring device.
- A "thickness" other than the above thickness is automatically measured under the condition of load: 0.098 N/cm² and pressure area: 2 cm² using an automatic thickness meter (KES-G5 handy compression measurement program).
- The "tensile strength" and the "tensile elongation (breaking elongation) refer to values measured by setting an initial chuck interval (distance between marked lines) to 50 mm and a tensile speed to 300 mm/min in accordance with JIS K7127: 1999 "Plastics-Determination of tensile properties-" except that the test piece has a rectangular shape of width 35 mm × length 80 mm. As a tensile testing machine, for example, AUTOGRAPH AGS-G100N manufactured by SHIMADZU CORPORATION can be used.
- The "stretching stress" refers to the tensile stress (N/35 mm) measured when stretching in the elastic region by a tensile test setting an initial chuck interval (distance between marked lines) to 50 mm and a tensile speed to 300 mm/min in accordance with JIS K7127: 1999 "Plastics-Determination of tensile properties-", and a degree of stretching can be appropriately determined depending on the test object. It is preferable that the test piece has a rectangular shape having a width of 35 mm and a length of 80 mm or more. However, when a test piece having a width of 35 mm may not be cut out, the test piece is created to have a width allowing cutting out, and a measured value is set to a value converted to have the width of 35 mm. In addition, even in a case in which the target region is small and sufficient test pieces may not be collected, when the magnitude of stretching stress is compared, even a suitably small test piece can be compared at least as long as test pieces of the same size are used. As a tensile testing machine, for example, AUTOGRAPH AGS-G100N manufactured by SHIMADZU CORPORATION can be used.
- The "unfolded state" refers to a flatly unfolded state without contraction or slack.
- Dimensions of each portion refer to dimensions in an unfolded state rather than the natural length state unless otherwise stated.
- When there is no description about an environmental condition in a test or measurement, it is presumed that the test or measurement is performed in a test room or a device in a standard state (temperature 23 ± 1°C, relative humidity 50 ± 2% in a test location).

### Industrial Applicability

As long as a stretchable region to which an elastic sheet stretchable structure can be applied is included, the invention can be used for elastic members in general disposable wearing articles such as various disposable diapers of a tape type, a pad type, etc., a sanitary napkin, a disposable wearing article for swimming or playing in the water, etc. in addition to the underpants-type disposable diaper as in the above example.

### Reference Signs List

- 10: Inner member
- 10B: Inner/outer fixing region
- 11: Top sheet
- 12: Liquid impervious sheet
- 13: Absorbent body
- 13N: Narrower portion
- 14: Package sheet
- 17: Non-absorbent body side portion
- 20: Outer member
- 20A: First sheet layer
- 20B: Second sheet layer
- 20C: Folded portion
- 20X: Elastic sheet stretchable structure
- 21: Side seal portion
- 23: Waist end portion region
- 24: Waist portion elastic member
- 25: Contraction pleats
- 29: Around-leg line
- 30: Elastic sheet
- 31: Joint hole
- 40: Sheet joined portion
- 51, 52: Non-joint band
- 51: First non-joint band
- 51d: First direction
- 51s: First interval
- 51w: First width
- 52: Second non-joint band
- 52d: Second direction
- 70: Non-stretchable region
- 80: Stretchable region
- 90: Three-dimensional gather
- 93: Fallen portion
- 94: Free portion
- 95: Gather sheet
- 96: Elastically stretchable gather member
- B: Back body
- ED: Stretchable direction
- F: Front body
- L: Intermediate portion
- LD: Front-back direction
- T: Lower torso portion
- WD: Width direction
- 71: Boundary
- ϕ: Reference bonding diameter

## Claims

1. An elastic member having an elastic sheet stretchable structure (20X) in which an elastic sheet (30) is interposed between a first sheet layer (20A) made of a nonwoven fabric and a second sheet layer (20B) made of a nonwoven fabric and the first sheet layer (20A) and the second sheet layer (20B) are welded through joint holes (31) penetrating the elastic sheet (30) at a plurality of sheet joined portions (40) arranged at intervals,
wherein the first sheet layer and the second sheet layer are formed of nonwoven fabrics having a fineness of 0.7 to 6 dtex and a basis weight of 10 to 25 g/m²,
a region having the elastic sheet stretchable structure (20X) includes a stretchable region (80) that contracts in an stretchable direction (ED) by contraction of the elastic sheet (30) and is extensible in the stretchable direction (ED),
the region having the elastic sheet stretchable structure (20X) includes sheet joined portions (40) having different shapes,
in all the sheet joined portions (40) in the region having the elastic sheet stretchable structure (20X), a reference bonding diameter **(ϕ)** is 0.25 to 0.5 mm, and a circumference length of the sheet joined portions is 1 to 15 times a length of a circumference of a circle whose diameter is the reference bonding diameter **(ϕ)**, and
the reference bonding diameter **(ϕ)**refers to a diameter of a largest inscribed circle inscribed in the outer shape of the sheet joined portion (40).

2. The elastic member according to claim 1,
wherein the region having the elastic sheet stretchable structure (20X) includes the stretchable region (80) and a non-stretchable region (70) provided on at least one side of the stretchable region (80) in the stretchable direction (ED),
an area ratio of the sheet joined portions (40) in the stretchable region (80) is 0.5 to 1 times an area ratio of the sheet joined portions (40) in the non-stretchable region (70), and
a boundary (71) between the stretchable region (80) and the non-stretchable region (70) has a shape continuously shifted only to one side in the stretchable direction (ED) from one end to the other end thereof.

3. An underpants-type disposable wearing article comprising:
an integrated outer member (20) from a front body (F) to a back body (B) or outer members (20) separately provided for the front body (F) and the back body (B);
an inner member (10) attached to an intermediate portion (L) of the outer member (20) in a width direction (WD), the inner member (20) extending to both front and back sides of a crotch portion;
side seal portions (21) in which both side portions of the outer member (20) in the front body (F) and both side portions of the outer member (20) in the back body (B) are bonded to each other, respectively; and
a waist opening and a pair of right and left leg openings,
wherein the outer member (20) in the front body (F) and the back body (B) has a lower torso portion (T) which is a range in a front-back direction (LD) corresponding to the side seal portions (21),
the outer member (20) in at least one of the front body (F) and the back body (B) has an intermediate portion (L) located on a center side of the lower torso portion (T) in the front-back direction (LD),
the intermediate portion (L) has edge portion regions along the leg openings,
the outer member (20) having the intermediate portion (L) is an elastic member having an elastic sheet stretchable structure (20X) of claim 1 from an inside of the intermediate portion (L) to an inside of the lower torso portion (T) in the front-back direction (LD) and between the side seal portions (21) in the width direction (WD) so that an stretchable direction (ED) of a stretchable region (80) thereof corresponds to the width direction (WD), and
a shape of the sheet joined portions (40) in the edge portion regions along the leg openings is different from a shape of the sheet joined portions (40) in other regions.

4. The disposable wearing article according to claim 3,
wherein the outer member (20) having the elastic sheet stretchable structure (20X) has a non-stretchable region (70) in an intermediate portion (L) in the width direction (WD) and a range in the width direction (WD) corresponding to a part between the non-stretchable region (70) and the side seal portions (21) is set as the stretchable region(80),
an area ratio of the sheet joined portions (40) in the stretchable region is 0.5 to 1 times an area ratio of the sheet joined portions (40) in the non-stretchable region, and
a boundary between the stretchable region and the non-stretchable region has a shape continuously shifted only to sides of the side seal portions (21) from an end of a waist opening side to a center of the disposable wearing article in the front-back direction (LD).

5. A method of manufacturing an elastic member according to claim 1, the method comprising:
a supplying process of interposing an elastic sheet (30) between a first sheet layer (20A) and a second sheet layer (20B) in a state of being stretched in an MD (machine direction); and
a bonding process of passing the first sheet layer (20A), the second sheet layer (20B), and the elastic sheet (30) interposed therebetween in a stretched state between an anvil roll having a plurality of projections arranged at intervals in a predetermined pattern on an outer peripheral surface and an ultrasonic horn facing the outer peripheral surface of the anvil roll, and welding the first sheet layer (20A) and the second sheet layer (20B) only at portions interposed between the plurality of projections and the ultrasonic horn to form sheet joined portions (40),
wherein sheet joined portions (40) having different reference bonding diameters **(ϕ)** are formed by one ultrasonic horn, and
in all the sheet joined portions (40) formed by the one ultrasonic horn, a reference bonding diameter **(ϕ)** is 0.25 to 0.5 mm, and a circumference length of the sheet joined portions is 1 to 15 times a length of a circumference of a circle whose diameter is the reference bonding diameter **(ϕ)**.

6. A method of manufacturing an elastic member according to claim 1, the method comprising:
a supplying process of interposing an elastic sheet (30) between a first sheet layer (20A) and a second sheet layer (20B) in a state of being stretched in an MD; and
a bonding process of passing the first sheet layer (20A), the second sheet layer (20B), and the elastic sheet (30) interposed therebetween in a stretched state between an anvil roll having a plurality of projections arranged at intervals in a predetermined pattern on an outer peripheral surface and an opposing roll facing the outer peripheral surface of the anvil roll, and welding the first sheet layer (20A) and the second sheet layer (20B) only at portions interposed between the plurality of projections and the opposing roll by the anvil roll and opposing roll that are heated to form sheet joined portions (40),
wherein sheet joined portions (40) having different reference bonding diameters **(ϕ)** are formed by a pair of anvil rolls and the opposing roll, and
in all the sheet joined portions (40) formed by the pair of anvil rolls and the opposing roll, a reference bonding diameter **(ϕ)** is 0.25 to 0.5 mm, and a circumference length of the sheet joined portions is 1 to 15 times a length of a circumference of a circle whose diameter is the reference bonding diameter **(ϕ).**

## Patentansprüche

1. Elastisches Element, das eine dehnbare elastische Lagenstruktur (20X) besitzt, bei der eine elastische Lage (30) zwischen einer ersten Lagenschicht (20A), die aus einem Vliesgewebe hergestellt ist, und einer zweiten Lagenschicht (20B), die aus einem Vliesgewebe hergestellt ist, angeordnet ist, und wobei die erste Lagenschicht (20A) und die zweite Lagenschicht (20B) durch Verbindungslöcher (31) verschweißt sind, die bei mehreren Lagenverbindungsabschnitten (40), die in Abständen angeordnet sind, die elastische Lage (30) durchdringen,
wobei die erste Lagenschicht und die zweite Lagenschicht aus Vliesgeweben gebildet sind, die eine Feinheit von 0,7 bis 6 dtex und ein Basisgewicht von 10 bis 25 g/m² haben,
wobei ein Bereich, der die dehnbare elastische Lagenstruktur (20X) hat, einen dehnbaren Bereich (80) umfasst, der sich durch die Kontraktion der elastischen Lage (30) in einer Dehnungsrichtung (ED) kontrahiert, und in der Dehnungsrichtung (ED) gedehnt werden kann,
wobei der Bereich, der die dehnbare elastische Lagenstruktur (20X) hat, Lagenverbindungsabschnitte (40) umfasst, die unterschiedliche Formen haben,
wobei in allen Lagenverbindungsabschnitten (40) in dem Bereich, der die dehnbare elastische Lagenstruktur (20X) hat, ein Referenzverbindungsdurchmesser (ϕ) 0,25 bis 0,5 mm beträgt, und eine Umfangslänge der Lagenverbindungsabschnitte das 1-fach bis 15-fache einer Länge eines Umfangs eines Kreises ist, dessen Durchmesser der Referenzverbindungsdurchmesser (ϕ) ist, und
wobei sich der Referenzverbindungsdurchmesser (ϕ) auf einen Durchmesser eines größten eingeschriebenen Kreises bezieht, der in die äußere Form des Lagenverbindungsabschnitts (40) eingeschrieben werden kann.

2. Elastisches Element nach Anspruch 1,
wobei der Bereich, der die dehnbare elastische Lagenstruktur (20X) hat, den dehnbaren Bereich (80) und einen nicht dehnbaren Bereich (70), der auf wenigstens einer Seite des dehnbaren Bereichs (80) in der Dehnungsrichtung (ED) vorgesehen ist, umfasst,
wobei ein Flächenverhältnis der Lagenverbindungsabschnitte (40) im dehnbaren Bereich (80) das 0,5-fache bis 1-fache eines Flächenverhältnisses der Lagenverbindungsabschnitte (40) im nicht dehnbaren Bereich (70) beträgt, und
eine Grenze (71) zwischen dem dehnbaren Bereich (80) und dem nicht dehnbaren Bereich (70) eine Form hat, die kontinuierlich nur zu einer Seite in der Dehnungsrichtung (ED) von einem Ende zum anderen Ende versetzt ist.

3. Tragbarer Wegwerfartikel des Unterhosentyps, der Folgendes umfasst:
ein integriertes äußeres Element (20) von einem vorderen Körper (F) zu einem hinteren Körper (B), oder äußere Elemente (20), die für den vorderen Körper (F) und den hinteren Körper (B) getrennt vorgesehen sind;
ein inneres Element (10), das an einem mittleren Abschnitt (L) des äußeren Elements (20) in einer Breitenrichtung (WD) befestigt ist, wobei sich das innere Element (20) zur Vorderseite und zur Rückseite eines Schrittabschnitts erstreckt;
seitliche Dichtungsabschnitte (21), wobei beide Seitenabschnitte des äu-ßeren Elements (20) im vorderen Körper (F) und beide Seitenabschnitte des äußeren Elements (20) im hinteren Körper (B) jeweils miteinander verbunden sind; und
eine Hüftöffnung und ein Paar rechter und linker Beinöffnungen,
wobei das äußere Element (20) im vorderen Körper (F) und im hinteren Körper (B) einen unteren Torsoabschnitt (T) hat, der ein Bereich in einer Richtung (LD) von vorn nach hinten ist, der den seitlichen Dichtungsabschnitten (21) entspricht,
wobei das äußere Element (20) im vorderen Körper (F) und/oder im hinteren Körper (B) einen mittleren Abschnitt (L) hat, der sich bei einer zentralen Seite des unteren Torsoabschnitts (T) in der Richtung (LD) von vorn nach hinten befindet,
wobei der mittlere Abschnitt (L) Kantenabschnittsbereiche längs der Beinöffnungen hat,
wobei das äußere Element (20), das den mittleren Abschnitt (L) hat, ein elastisches Element ist, das eine dehnbare elastische Lagenstruktur (20X) nach Anspruch 1 von der Innenseite des mittleren Abschnitts (L) zur Innenseite des unteren Torsoabschnitts (T) in der Richtung (LD) von vorn nach hinten und zwischen den seitlichen Dichtungsabschnitten (21) in der Breitenrichtung (WD) hat, so dass eine Dehnungsrichtung (ED) eines dehnbaren Bereichs (80) der Breitenrichtung (WD) entspricht, und
wobei sich eine Form der Lagenverbindungsabschnitte (40) in den Kantenabschnittsbereichen längs der Beinöffnungen von einer Form der Lagenverbindungsabschnitte (40) in anderen Bereichen unterscheidet.

4. Tragbarer Wegwerfartikel nach Anspruch 3,
wobei das äußere Element (20), das die dehnbare elastische Lagenstruktur (20X) hat, einen nicht dehnbaren Bereich (70) in einem mittleren Abschnitt (L) in der Breitenrichtung (WD) hat, und wobei ein Bereich in der Breitenrichtung (WD), der einem Teil zwischen dem nicht dehnbaren Bereich (70) und den seitlichen Dichtungsabschnitten (21) entspricht, als der dehnbare Bereich (80) festgelegt ist,
wobei ein Flächenverhältnis der Lagenverbindungsabschnitte (40) im dehnbaren Bereich das 0,5-fache bis 1-fache eines Flächenverhältnisses der Lagenverbindungsabschnitte (40) im nicht dehnbaren Bereich beträgt, und
eine Grenze zwischen dem dehnbaren Bereich und dem nicht dehnbaren Bereich eine Form hat, die kontinuierlich nur zu den Seiten der seitlichen Dichtungsabschnitte (21) von einem Ende einer Hüftöffnungsseite zur Mitte des tragbaren Wegwerfartikels in der Richtung (LD) von vorn nach hinten versetzt ist.

5. Verfahren zum Herstellen eines elastischen Elements nach Anspruch 1, wobei das Verfahren die folgenden Schritte umfasst:
einen Zuführvorgang, um eine elastische Lage (30) zwischen einer ersten Lagenschicht (20A) und einer zweiten Lagenschicht (20B) in einem Zustand, in dem sie in einer MD-Richtung (Maschinenrichtung) gedehnt ist, anzuordnen; und
einen Verbindungsvorgang, bei dem die erste Lagenschicht (20A), die zweite Lagenschicht (20B) und die elastische Lage (30), die in einem gedehnten Zustand dazwischen angeordnet ist, zwischen einer Anpresswalze, die mehrere Vorsprünge hat, die in Abständen in einem festgelegten Muster auf einer Außenumfangsfläche angeordnet sind, und einem Ultraschallhorn, das zur Außenumfangsfläche der Anpresswalze zeigt, geführt werden, und die erste Lagenschicht (20A) und die zweite Lagenschicht (20B) nur bei Abschnitten, die zwischen den mehreren Vorsprüngen und dem Ultraschallhorn angeordnet sind, verschweißt werden, um Lagenverbindungsabschnitte (40) zu bilden,
wobei Lagenverbindungsabschnitte (40), die unterschiedliche Referenzverbindungsdurchmesser (ϕ) haben, durch ein Ultraschallhorn gebildet werden, und
wobei bei allen Lagenverbindungsabschnitten (40), die durch das eine Ultraschallhorn gebildet werden, ein Referenzverbindungsdurchmesser (ϕ) 0,25 bis 0,5 mm beträgt, und eine Umfangslänge der Lagenverbindungsabschnitte das 1-fache bis 15-fache einer Länge eines Umfangs eines Kreises beträgt, dessen Durchmesser der Referenzverbindungsdurchmesser (ϕ) ist.

6. Verfahren zum Herstellen eines elastischen Elements nach Anspruch 1, wobei das Verfahren die folgenden Schritte umfasst:
einen Zuführvorgang, um eine elastische Lage (30) zwischen einer ersten Lagenschicht (20A) und einer zweiten Lagenschicht (20B) in einen Zustand, in dem sie in einer MD-Richtung gedehnt ist, anzuordnen; und
einen Verbindungsvorgang, bei dem die erste Lagenschicht (20A), die zweite Lagenschicht (20B) und die elastische Lage (30), die in einem gedehnten Zustand dazwischen angeordnet ist, zwischen einer Anpresswalze, die mehrere Vorsprünge hat, die in Abständen in einem festgelegten Muster auf einer Außenumfangsfläche angeordnet sind, und einer gegenüberliegenden Walze, die zur Außenumfangsfläche der Anpresswalze zeigt, geführt werden, und die erste Lagenschicht (20A) und die zweite Lagenschicht (20B) nur bei Abschnitten, die zwischen den mehreren Vorsprüngen und der gegenüberliegenden Walze angeordnet sind, durch die Anpresswalze und die gegenüberliegende Walze, die geheizt sind, verschweißt werden, um die Lagenverbindungsabschnitte (40) zu bilden,
wobei die Lagenverbindungsabschnitte (40), die unterschiedliche Referenzverbindungsdurchmesser (ϕ) haben, durch ein Paar Anpresswalzen und die gegenüberliegende Walze gebildet werden, und
wobei in allen Lagenverbindungsabschnitte (40), die durch das Paar Anpresswalzen und die gegenüberliegende Walze gebildet werden, ein Referenzverbindungsdurchmesser (ϕ) 0,25 bis 0,5 mm beträgt, und eine Umfangslänge der Lagenverbindungsabschnitte das 1-fache bis 15-fache einer Länge eines Umfangs eines Kreises beträgt, dessen Durchmesser der Referenzverbindungsdurchmesser (ϕ) ist.

## Revendications

1. Élément élastique ayant une structure étirable à feuille élastique (20X) dans laquelle une feuille élastique (30) est interposée entre une première couche en feuille (20A) faite d'une étoffe non tissée et une seconde couche en feuille (20B) faite d'une étoffe non tissée, et la première couche en feuille (20A) et la seconde couche en feuille (20B) sont soudées au moyen de trous de jonction (31) qui pénètrent dans la feuille élastique (30) au niveau d'une pluralité de portions jointes de feuilles (40) agencées à des intervalles,
dans lequel la première couche en feuille et la seconde couche en feuille sont formées d'étoffes non tissées ayant une finesse de 0,7 à 6 dtex et un poids de base de 10 à 25 g/m²,
une région ayant la structure étirable à feuille élastique (20X) inclut une région étirable (80) qui se contracte dans une direction étirable (ED) par contraction de la feuille élastique (30) et qui est extensible dans la direction étirable (ED),
la région ayant la structure étirable à feuille élastique (20X) inclut des portions jointes de feuilles (40) ayant des formes différentes,
dans la totalité des portions jointes de feuilles (40) dans la région ayant la structure étirable à feuille élastique (20X), un diamètre de liaison de référence (cp) est de 0,25 à 0,5 mm, et une longueur de circonférence des portions jointes de feuilles est de 1 à 15 fois une longueur d'une circonférence d'un cercle dont le diamètre est le diamètre de liaison de référence (cp), et
le diamètre de liaison de référence (cp) se réfère à un diamètre d'un cercle inscrit le plus grand, inscrit dans la forme extérieure de la portion jointe de feuilles (40).

2. Élément élastique selon la revendication 1,
dans lequel la région ayant la structure étirable à feuille élastique (20X) inclut la région étirable (80) et une région non étirable (70) prévue sur au moins un côté de la région étirable (80) dans la direction étirable (ED),
un rapport d'aire des portions jointes de feuilles (40) dans la région étirable (80) est de 0,5 à 1 fois un rapport d'aire des portions jointes de feuilles (40) dans la région non étirable (70), et
une frontière (71) entre la région étirable (80) et la région non étirable (70) a une forme décalée en continu uniquement jusqu'à un côté dans la direction étirable (ED) depuis une extrémité jusqu'à l'autre extrémité de celle-ci.

3. Article à porter jetable du type couche-culotte comprenant :
un élément extérieur intégré (20) depuis un corps avant (F) jusqu'à un corps arrière (B) ou des éléments extérieurs (20) prévus séparément pour le corps avant (F) et le corps arrière (B) ;
un élément intérieur (10) fixé à une portion intermédiaire (L) de l'élément extérieur (20) dans une direction de la largeur (WD), l'élément intérieur (20) s'étendant jusqu'aux deux côtés avant et arrière d'une portion d'entrejambe ;
des portions d'étanchéité latérales (21) dans lesquelles les deux portions latérales de l'élément extérieur (20) dans le corps avant (F) et les deux portions latérales de l'élément extérieur (20) dans le corps arrière (B) sont liées l'une à l'autre, respectivement ; et
une ouverture de taille et une paire d'ouvertures de jambe de droite et de gauche,
dans lequel l'élément extérieur (20) dans le corps avant (F) et le corps arrière (B) a une portion de torse inférieure (T) qui est une plage dans une direction avant/arrière (LD) correspondant aux portions d'étanchéité latérales (21),
l'élément extérieur (20) dans l'un au moins du corps avant (F) et du corps arrière (B) a une portion intermédiaire (L) située sur un côté central de la portion de torse inférieure (T) dans la direction avant/arrière (LD),
la portion intermédiaire (L) a des régions de portions de bord le long des ouvertures de jambe,
l'élément extérieur (20) ayant la portion intermédiaire (L) est un élément élastique ayant une structure étirable à feuille élastique (20X) selon la revendication 1 depuis un intérieur de la portion intermédiaire (L) jusqu'à un intérieur de la portion de torse inférieure (T) dans la direction avant/arrière (LD) et entre les portions d'étanchéité latérales (21) dans la direction de la largeur (WD) de telle sorte qu'une direction étirable (ED) d'une région étirable (80) de celle-ci correspond à la direction de la largeur (WD), et
une forme des portions jointes de feuilles (40) dans les régions de portions de bord le long des ouvertures de jambe est différente d'une forme des portions jointes de feuilles (40) dans d'autres régions.

4. Article à porter jetable selon la revendication 3,
dans lequel l'élément extérieur (20) ayant la structure étirable à feuille élastique (20X) a une région non étirable (70) dans une portion intermédiaire (L) dans la direction de la largeur (WD) et une plage dans la direction de la largeur (WD) correspondant à une partie entre la région non étirable (70) et les portions d'étanchéité latérales (21) est établie comme la région étirable (80),
un rapport d'aire des portions jointes de feuilles (40) dans la région étirable est de 0,5 à 1 fois un rapport d'aire des portions jointes de feuilles (40) dans la région non étirable, et
une frontière entre la région étirable et la région non étirable a une forme décalée en continu uniquement jusqu'à des côtés des portions d'étanchéité latérales (21) depuis une extrémité d'un côté d'ouverture de taille jusqu'à un centre de l'article à porter jetable dans la direction avant/arrière (LD).

5. Procédé de fabrication d'un élément élastique selon la revendication 1, le procédé comprenant :
une étape d'alimentation consistant à interposer une feuille élastique (30) entre une première couche en feuille (20A) et une seconde couche en feuille (20B) dans un état étiré dans une direction de machine (MD) ; et
une étape de liaison consistant à faire passer la première couche en feuille (20A), la seconde couche en feuille (20B) et la feuille élastique (30) interposée entre celles-ci dans un état étiré entre un cylindre enclume ayant une pluralité de projections agencées à des intervalles dans un motif prédéterminé sur une surface périphérique extérieure et un cornet à ultrasons faisant face à la surface périphérique extérieure du cylindre enclume, et à souder la première couche en feuille (20A) et la seconde couche en feuille (20B) uniquement au niveau de portions interposées entre la pluralité de protection et le cornet à ultrasons pour former des portions jointes de feuilles (40),
dans lequel les portions jointes de feuilles (40) ayant des diamètres de liaison de référence (cp) différents sont formées par un cornet à ultrasons, et dans la totalité des portions jointes de feuilles (40) formées par ledit un cornet à ultrasons, un diamètre de liaison de référence (cp) est de 0,25 à 0,5 mm, et une longueur de circonférence des portions jointes de feuilles est de 1 à 15 fois une longueur d'une circonférence d'un cercle dont le diamètre est le diamètre de liaison de référence (cp).

6. Procédé de fabrication d'un élément élastique selon la revendication 1, le procédé comprenant :
une étape d'alimentation consistant à interposer une feuille élastique (30) entre une première couche en feuille (20A) et une seconde couche en feuille (20B) dans un état étiré dans une direction de machine (MD) ; et
une étape de liaison consistant à faire passer la première couche en feuille (20A), la seconde couche en feuille (20B) et la feuille élastique (30) interposée entre celles-ci dans un état étiré entre un cylindre enclume ayant une pluralité de projections agencées à des intervalles dans un motif prédéterminé sur une surface périphérique extérieure et un cylindre opposé faisant face à la surface périphérique extérieure du cylindre enclume, et à souder la première couche en feuille (20A) et la seconde couche en feuille (20B) uniquement au niveau de portions interposées entre la pluralité de protections et le cylindre opposé via le cylindre enclume et le cylindre opposé qui sont chauffés pour former des portions jointes de feuilles (40),
dans lequel les portions jointes de feuilles (40) ayant des diamètres de liaison de référence (cp) différents sont formées par une paire de cylindres enclumes et le cylindre opposé, et
dans la totalité des portions jointes de feuilles (40) formées par la paire de cylindres enclumes et le cylindre opposé, un diamètre de liaison de référence (cp) est de 0,25 à 0,5 mm, et une longueur de circonférence des portions jointes de feuilles est de 1 à 15 fois une longueur d'une circonférence d'un cercle dont le diamètre est le diamètre de liaison de référence (cp).
